# EUROPEAN PATENT SPECIFICATION

(11) **EP 0 432 677 B1**
(45) Date of publication and mention of the grant of the patent: **06.03.1996**
(21) Application number: 90123671.1
(22) Date of filing: 10.12.1990
(51) Int. Cl.: C07D 487/04, A61K 31/505

(54) **N-(pyrrolo[2,3-d]pyrimidin-3-ylacyl)-glutamic acid derivatives**
N-(Pyrrolo(2-3-d)pyrimidin-3-ylacyl)-Glutaminsäurederivate
Dérivés de l'acide N-(pyrrolo(2,3-d)pyrimidine-3-ylacyl)-glutamique

(30) Priority: 11.12.1989 US 448742; 08.02.1990 US 479655; 24.05.1990 US 528805; 24.05.1990 US 528155
(43) Date of publication of application: 19.06.1991
(73) Proprietor: THE TRUSTEES OF PRINCETON UNIVERSITY, Princeton New Jersey 08544 (US); ELI LILLY AND COMPANY, Indianapolis, Indiana 46285 (US)
(72) Inventor: Taylor, Edward C., Princeton, New Jersey 08544 (US); Kuhnt, Dietmar G., W-5090 Leverkusen 1 (DE); Shih, Chuan, Indianapolis, Indiana 46256 (US); Grindey, Gerald B., Indianapolis, Indiana 46502 (US)
(74) Representative: Minoja, Fabrizio

(56) References cited:
- EP-A- 0 334 636
- EP-A- 0 334 636
- EP-A- 0 343 801
- US-A- 4 818 819
- US-A- 4 883 799

## Description

The present invention pertains to glutamic acid derivatives having the formula: 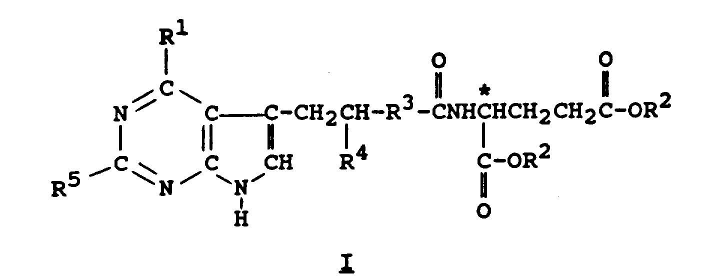 in which:
- R¹: is -OH or -NH₂;
- R: is hydrogen or a pharmaceutically acceptable cation;
- R³: is 1,4-phenylene or 1,3-phenylene unsubstituted or substituted with chloro, fluoro, methyl, methoxy, or trifluoromethyl; thienediyl unsubstituted or substituted with chloro, fluoro, methyl, methoxy, or trifluoromethyl; cyclohexanediyl; or a straight or branched divalent aliphatic group of from 2 to 4 carbon atoms;
- R⁴: is hydrogen;
- R⁵: is hydrogen, alkyl of 1 to 6 carbon atoms, or amino; and the configuration about the carbon atom designated * is S.

The compounds of this invention are herein described as embodying the pyrrolo[2,3-d]pyrimidine heterocyclic ring system which ring system is numbered as follows:

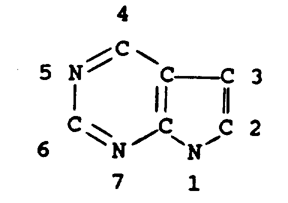

It will be appreciated that the pyrrolo[2,3-d]pyrimidines as depicted by Formula I are the tautomeric equivalent of the corresponding 5-H-6-oxo or 5-H-6-imino structures. Unless otherwise indicated, for simplicity's sake the compounds are depicted herein and named using the 6-hydroxy and 6-amino convention, it being understood the 5-H-6-oxo and 5-H-6-imino structures are fully equivalent.

The compounds of Formula I have an inhibitory effect on one or more enzymes which utilize folic acid, and in particular metabolic derivatives of folic acid, as a substrate. The compounds appear to be particularly active as inhibitors of thymidylate synthetase, which catalyses the methylation of deoxyuridylic acid to deoxythymidylic acid utilizing N⁵,N¹⁰-methylidenetetrahydrofolate as a coenzyme. The compounds thus can be used, alone or in combination, to inhibit the growth of those neoplasms which otherwise depend upon the inhibited enzyme.

The invention also pertains to the pharmaceutically acceptable salts of the compounds of Formula I, to processes for the preparation of these compounds and their salts, to chemical intermediates useful in preparation of these compounds, to a method of combatting neoplastic growth in a mammal, and to pharmaceutical compositions containing these compounds or their salts.

A first group of useful chemical intermediates, which can be converted directly to the desired final compounds of Formula I through removal of protecting groups, are compounds of the formula: 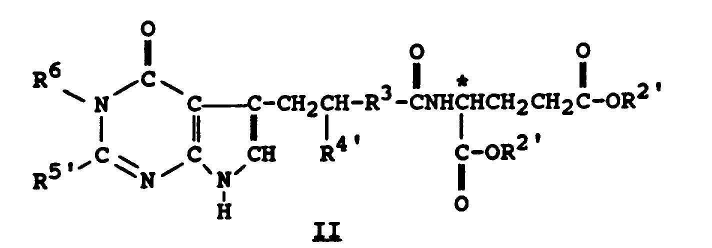 in which:
- R³: is as defined above;
- R^{2'}: is hydrogen or a carboxy protecting group;
- R^{4'}: is hydrogen;
- R^{5'}: is hydrogen, alkyl, amino, or amino carrying a protecting group, and
- R⁶: is hydrogen or alkanoyloxy;
at least one of R^{2'} and R^{5'} being a carboxy protecting group, a hydroxy protecting group, or an amino protecting group, respectively.

The compounds of Formula I can be employed in the form of the free dicarboxylic acid, in which case both R groups are hydrogen. Alternatively, the compounds often can be employed advantageously in the form of a pharmaceutically acceptable salt, in which case one or both R groups are a pharmaceutically acceptable cation. Such salt forms, including hydrates thereof, are often crystalline and advantageous for forming solutions or formulating pharmaceutical compositions. Pharmaceutically acceptable salts with bases include those formed from the alkali metals, alkaline earth metals, non-toxic metals, ammonium, and mono-, di- and trisubstituted amines, such as for example the sodium, potassium, lithium, calcium, magnesium, aluminum, zinc, ammonium, trimethylammonium, triethanolammonium, pyridinium, and substituted pyridinium salts. The mono and disodium salts, particularly the disodium salt, are advantageous.

The group R³ is a divalent group having at least two carbon atoms between the carbon atoms carrying the free valence bonds. R³ for example can be a 1,4-phenylene or 1,3-phenylene ring which is unsubstituted or optionally substituted with chloro, fluoro, methyl, methoxy, or trifluoromethyl.

Alternatively, R³ can be a thienediyl group, that is, a thiophene ring from which two hydrogen atoms have been removed from two ring carbon atoms, as for example the thiene-2,5-diyl, thiene-3,5-diyl, thiene-2,4-diyl, and thiene-3,4-diyl ring systems, which ring systems can be unsubstituted or substituted with chloro, fluoro, methyl, methoxy, or trifluoromethyl. It will be appreciated that whereas in the abstract the thiene-3,5-diyl system is the equivalent of the thiene-2,4-diyl system, the two terms are utilized herein to denote the two isomeric forms resulting from the orientation of the thiophene ring within the remainder of the molecule: e.g. the structure in which the depicted carboxy group adjacent to R³ is in the 2-position of the thiophene ring and that in which the depicted carboxy group adjacent to R³ is in the 3-position of the thiophene ring.

Alternatively, R³ can be a cyclohexanediyl group, namely a divalent cycloalkane group of 6 carbon atoms such as cyclohexane-1,3-diyl and cyclohexane-1,4-diyl.

Alternatively, R³ can be a alkanediyl, namely a straight or branched divalent aliphatic group of from 2 to 4 carbon atoms such as ethano, trimethylene, tetramethylene, propane-1,2-diyl, propane-2,3-diyl, butane-2,3-diyl, butane-1,3-diyl, and butane-2,4-diyl. It again will be appreciated that whereas in the abstract propane-1,2-diyl is the equivalent of propane-2,3-diyl, and butane-1,3-diyl the equivalent of butane-2,4-diyl, the two terms are utilized herein to denote the two isomeric forms resulting from the orientation of an unsymmetrical alkanediyl chain with respect to the remainder of the molecule.

The protecting groups designated by R^{2'} and R^{5'} and utilized herein denote groups which generally are not found in the final therapeutic compounds but which are intentionally introduced at a stage of the synthesis in order to protect groups which otherwise might react in the course of chemical manipulations, thereafter being removed at a later stage of the synthesis. Since compounds bearing such protecting groups thus are of importance primarily as chemical intermediates (although some derivatives also exhibit biological activity), their precise structure is not critical. Numerous reactions for the formation and removal of such protecting groups are described in a number of standard works including, for example, "Protective Groups in Organic Chemistry", Plenum Press, London and New York, 1973; Greene, Th. W. "Protective Groups in Organic Synthesis", Wiley, New York, 1981; "The Peptides", Vol. I, Schröder and Lubke, Academic Press, London and New York, 1965; "Methoden der organischen Chemie", Houben-Weyl, 4th Edition, Vol.15/I, Georg Thieme Verlag, Stuttgart 1974, the disclosures of which are incorporated herein by reference.

With respect to R^{2'}, a carboxy group can be protected as an ester group which is selectively removable under sufficiently mild conditions not to disrupt the desired structure of the molecule, especially a lower alkyl ester of 1 to 12 carbon atoms such as methyl or ethyl and particularly one which is branched at the 1-position such as t-butyl; and such lower alkyl ester substituted in the 1- or 2-position with (i) lower alkoxy, such as for example, methoxymethyl, 1-methoxyethyl, and ethoxymethyl, (ii) lower alkylthio, such as for example methylthiomethyl and 1-ethylthioethyl; (iii) halogen, such as 2,2,2-trichloroethyl, 2-bromoethyl, and 2-iodoethoxycarbonyl; (iv) one or two phenyl groups each of which can be unsubstituted or mono-, di- or tri-substituted with, for example lower alkyl such as tert.-butyl, lower alkoxy such as methoxy, hydroxy, halo such as chloro, and nitro, such as for example, benzyl, 4-nitrobenzyl, diphenylmethyl, di-(4-methoxyphenyl)methyl; or (v) aroyl, such as phenacyl. A carboxy group also can be protected in the form of an organic silyl group such as trimethylsilylethyl or trilower alkylsilyl, as for example trimethylsilyloxycarbonyl.

With respect to R^{5'}, an amino group can be protected as an amide utilizing an acyl group which is selectively removable under mild conditions, especially formyl, a lower alkanoyl group which is branched α to the carbonyl group, particularly tertiary alkanoyl such as pivaloyl, or a lower alkanoyl group which is substituted in the position α to the carbonyl group, as for example trifluoroacetyl.

Preferred compounds of Formula I are those wherein R⁵ is amino or hydrogen. Within this class, R¹ preferably is hydroxy and R³ is 1,4-phenylene. Also preferred within this class are the compounds in which R¹ is hydroxy and R³ is thienediyl.

The compounds of this invention can be prepared according to a first process through catalytic hydrogenation of a compound of the formula: 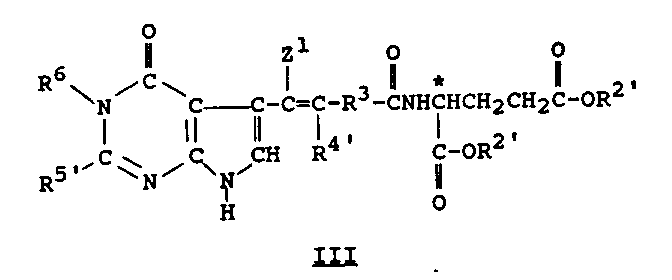 in which:
- Z¹: is hydrogen, or Z¹ taken together with R^{4'} is a carbon-carbon bond;
- R^{2'}: is hydrogen or a carboxy protecting group;
- R³ and R⁶: are as defined above;
- R^{4'},: when taken independently of Z¹, is hydrogen;
- R^{5'}: is hydrogen, alkyl of 1 to 6 carbon atoms, amino, or an amino protecting group.

Suitable hydrogenation catalysts include noble metals and noble metal oxides such as palladium or platinum oxide, rhodium oxide, and the foregoing on a support such as carbon or calcium oxide.

When in Formula III, Z¹ taken together with R^{4'} is a carbon-carbon bond, that is, when a triple bond is present between the two carbon atoms to which Z¹ and R^{4'} are bound, R^{4'} in the hydrogenation product will be hydrogen. Absent any chirality in R³ (or any protecting group encompassed by R^{2'} and/or R^{5'}), the hydrogenation product will be a single enantiomer having the S-configuration about the carbon atom designated *. This also is true when Z¹ and R^{4'} are each hydrogen, that is, when a double bond is present between the two carbon atoms to which Z¹ and R^{4'} are bound.

The protecting groups encompassed by R^{2'}, R^{5'} and/or R⁶ can be removed following hydrogenation through acidic or basic hydrolysis, as for example with sodium hydroxide to cleave R^{2'} or R^{5'} protecting groups, thereby yielding the compounds of Formula I. Methods of removing the various protective groups are described in the standard references noted above and incorporated herein by reference.

Compounds of Formula III can be prepared utilizing procedures analogous to those described in U.S. Patent No. 4,818,819, utilizing however the corresponding halogenated pyrrolo[2,3-d]pyrimidine. Thus a pyrrolo[2,3-d]pyrimidine of the formula: 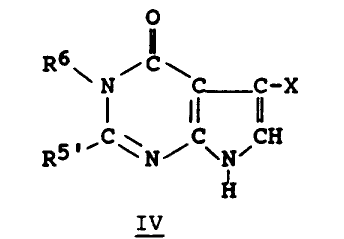 in which X is bromo or iodo and R^{5'} and R⁶ are as herein defined, is allowed to react with an unsaturated compound of the formula: 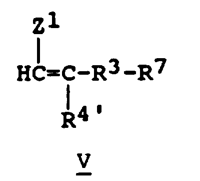 in which Z¹, R³, and R^{4'} are as herein defined and R⁷ is 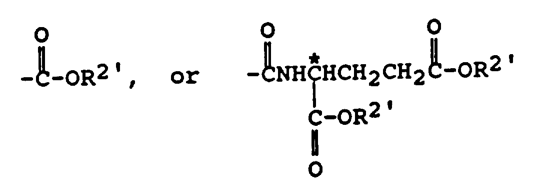 in which R^{2'} is as herein defined, in the presence of a palladium/trisubstituted phosphine catalyst of the type described in U.S. Patent No. 4,818,819, the disclosure of which is incorporated herein by reference.

When R⁷ is -CONHCH(COOR^{2'})CH₂CH₂COOR^{2'}, the product of this coupling reaction is hydrogenated, and any protecting group removed, as described above.

Alternatively, a compound of Formula IV is allowed to react with a compound of the formula: 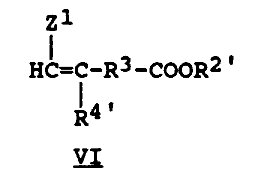 in which Z¹, R^{2'}, R³, and R^{4'} are as herein defined in the presence of a palladium/trisubstituted phosphine catalyst of the type described in U.S. Patent No. 4,818,819 to yield an intermediate of the formula:

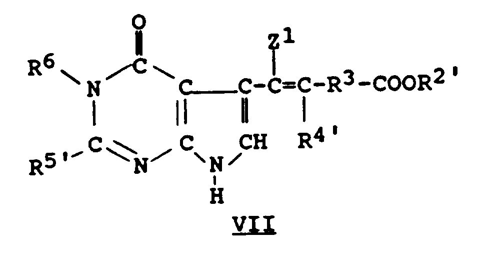

The product of Formula VII then can be hydrogenated, hydrolysed to remove the R^{2'} and R⁶ protecting groups, and, optionally with intermediate protection of any amino group encompassed by R^{5'}, coupled with a protected glutamic acid derivative in the manner described in U.S. Patent No. 4,684,653 using conventional condensation techniques for forming peptide bonds such as DCC or diphenylchlorophosphonate, following which the protecting groups are removed.

In a further variant, compounds of Formula III can be prepared utilizing the procedures described in U.S. Patent No. 4,818,819. Thus a compound of the formula: 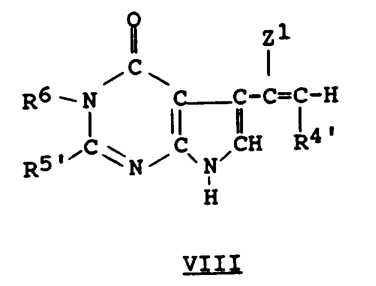 in which Z¹, R^{4'}, R^{5'} and R⁶ are as herein defined, is allowed to react with a compound of the formula:

X-R³-R⁷ IX

in which X, R³, and R⁷ are as herein defined, in the presence of a palladium/trisubstituted phosphine catalyst of the type described in U.S. Patent No. 4,818,819.

Compounds of Formula VIII also can be obtained by the methods of U.S. Patent No. 4,818,819 by treating a compound of Formula IV with an unsaturated compound of the formula:

H-C≡C-R^{4''} X

in which R^{4''} is a trisubstituted silyl group in the presence of a palladium/trisubstituted phosphine catalyst of the type discussed above.

Although not always the case, the compounds of Formula IV in which R⁶ is hydrogen can tend to be somewhat insoluble in solvents suitable for the reaction described in U.S. Patent No. 4,818,819. In such instances, the compounds of Formula IV in which R⁶ is hydrogen can be first treated with sodium hydride and a suitable alkyl alkanoate (such as chloromethyl pivalate) to introduce an alkanoyloxy group in the 5-position and increase solubility.

A useful subclass of compounds useful both as intermediates and for their effect on enzymes are derivatives of Formula XI and XII lacking the glutamic acid side-chain: 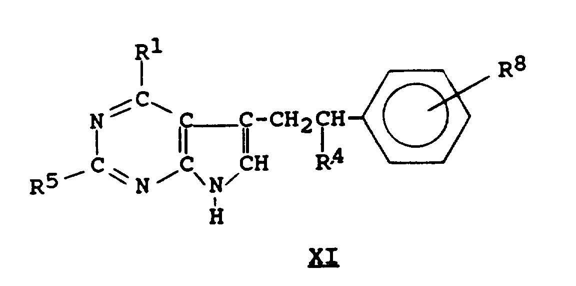 and 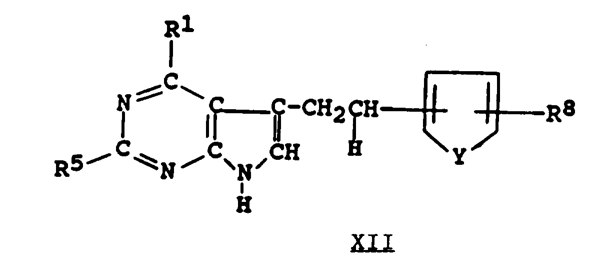 in which:
- R¹: is -OH or -NH₂;
- R⁴: is hydrogen;
- R⁵: is hydrogen, alkyl of 1 to 6 carbon atoms, or amino,
- R⁸: is hydrogen, chloro, fluoro, methyl, methoxy, trifluoromethyl, or carboxy;
- Y: is -S- or -O-; and
the pharmaceutically acceptable salts thereof.

Compounds of Formulas XI and XII are obtained by allowing a compound of Formula VII to react with a compound of the formula: 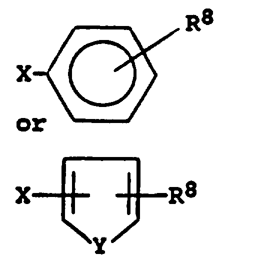 in which X, Y, and R⁸ are as herein defined by the methods of U.S. Patent No. 4,818,819, namely in the presence of a palladium/trisubstituted phosphine catalyst, with the resulting coupled product being hydrogenated and hydrolysed to remove the R^{2'} protecting group. Typical compounds of Formulas XI and XII are 3-(2-phenyl-3-hydroxypropyl)-4-hydroxy-6-amino-pyrrolo[2,3-d]pyrimidine, 3-[2-(thien-2-yl)ethyl]-4-hy-droxy-6-aminopyrrolo[2,3-d]pyrimidine, 3-[2-(thien-2-yl)ethyl]-4-hydroxypyrrolo[2,3-d]pyrimidine, 3-[2-(thien-2-yl)ethyl]-4-hydroxy-6-methylpyrrolo[2,3-d]py-rimidine, 3-[2-(thien-3-yl)ethyl]-4-hydroxy-6-amino-pyrrolo[2,3-d]pyrimidine, 3-[2-(thien-3-yl)ethyl]-4-hydroxypyrrolo[2,3-d]pyrimidine, and 3-[2-(thien-3-yl)-ethyl]-4-hydroxy-6-methylpyrrolo[2,3-d]pyrimidine.

As discussed above, the compounds of this invention can be prepared utilizing the palladium catalyzed coupling of various unsaturated compounds described in U.S. Patent No. 4,818,819 and the glutamic acid coupling reactions described in U.S. Patent No. 4,684,653, substituting the appropriate pyrrolo[2,3-d]pyrimidine for the pyrido[2,3-d]pyrimidine therein disclosed. The pyrrolo[2,3-d]pyrimidine intermediates of Formula IV above can be obtained by treating a compound of the formula: 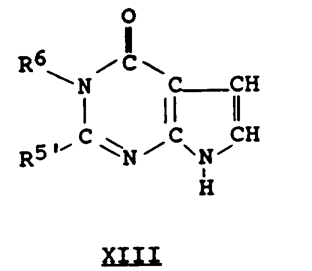 in which R^{5'} and R⁶ are as herein defined with N-iodosuccinimide to yield the corresponding 2,3-diiodopyrrolo[2,3-d]pyrimidine which then is treated with zinc and acetic acid to remove selectively the iodine atom in the 2-position, yielding the corresponding 3-iodopyrrolo[2,3-d]pyrimidine of Formula IV.

According to the foregoing processes, compounds of Formula II in which R¹ is -OH are obtained. When a compound of Formula I in which R¹ is -NH₂ is desired, a compound in which R¹ is -OH can be treated with 1,2,4-triazole and (4-chlorophenyl)dichlorophosphate and the product of this reaction then treated with concentrated ammonia.

As noted, the compounds of this invention have an effect on one or more enzymes which utilize folic acid, and in particular metabolic derivatives of folic acid, as a substrate. For example, N-{4-[2-(4-hydroxy-6-aminopyrrolo[2,3-d]pyrimidin-3-yl)ethyl]benzoyl}-L-glutamic acid demonstrates potent inhibitory effects against growth of human T-cell derived lyphoblastic leukemia cells (CCRF-CEM), exhibiting an IC₅₀ of 0.004 µ/ml. Cytotoxicity is not reversed by addition of purines such as hypoxanthine or by addition of aminoimidazolecarboxamide but is reversed by addition of thymidine, indicating specific inhibition in the tymidylate cycle and not in de novo purine synthesis. The compounds can be used, under the supervision of qualified professionals, to inhibit the growth of neoplasms including choriocarcinoma, leukemia, adenocarcinoma of the female breast, epidermid cancers of the head and neck, squamous or small-cell lung cancer, and various lymphosarcomas. The compounds can also be used to treat mycosis fungoides and psoriasis.

The compounds can be administered orally but preferably are administered parenterally, alone or in combination with other therapeutic agents including other anti-neoplastic agents, steroids, etc., to a mammal suffering from neoplasm and in need of treatment. Parenteral routes of administration include intramuscular, intrathecal, intravenous and intra-arterial. Dosage regimens must be titrated to the particular neoplasm, the condition of the patient, and the response but generally doses will be from about 10 to about 100 mg/day for 5-10 days or single daily administration of 250-500 mg, repeated periodically; e.g. every 14 days. While having a low toxicity as compared to other antimetabolites now in use, a toxic response often can be eliminated by either or both of reducing the daily dosage or administering the compound on alternative days or at longer intervals such as every three days. Oral dosage forms include tablets and capsules containing from 1-10 mg of drug per unit dosage. Isotonic saline solutions containing 20-100 mg/ml can be used for parenteral administration.

The following examples will serve to further illustrate the invention. In the NMR data, "s" denotes singlet, "d" denotes doublet, "t" denotes triplet, "q" denotes quartet, "m" denotes multiplet, and "br" denotes a broad peak.

### Example 1

### 3-IODO-4-HYDROXY-6-PIVALOYLAMINOPYRROLO[2,3-d]PYRIMIDINE

A mixture of 3.0 g (0.02 mole) of 4-hydroxy-6-aminopyrrolo[2,3-d]pyrimidine and 8.4 g (0.07 mol) of pivaloyl chloride in 40 mL of pyridine is stirred for 30 minutes at from 80 to 90°C, the mixture then evaporated to dryness, and the residue dissolved in 30 mL of methanol. Addition of 10% aqueous ammonia yields 4.2 g (89%) of 4-hydroxy-6-pivaloylaminopyrrolo[2,3-d]pyrimidine which can be further purified by chromatography through silica gel, eluting with 8% methanol in methylene chloride. mp 295°C. ¹NMR (d₆-DMSO) δ 1.20(s, 9H), 6.37(d, J = 3.4Hz, 1H), 6.92(d, J = 3.4Hz, 1H), 10.78 (s, 1H), 11.56 (s, 1H), 11.82 (s, 1H). Anal. Calc. for C₁₁H₁₄N₄O₂: C, 56.40; H, 6.02; N, 23.92. Found: C, 56.16; H, 6.01; N, 23.67.

To a mixture of 4.7 g (20 mmol) of 4-hydroxy-6-pivaloylaminopyrrolo[2,3-d]pyrimidine in 200 mL of dimethylformamide are added 9.9 g (44 mmol) of N-iodosuccinamide. The mixture is stirred at ambient temperature in the dark for 18 hours. Most of the dimethylformamide is removed by evaporation and the residual slurry poured into 300 mL of water. The resulting solid is collected by filtration and dried under vacuum over phosphorus pentoxide to yield 2,3-diiodo-4-hydroxy-6-pivaloylaminopyrrolo[2,3-d]pyrimidine which can be purified further by chromatography over silica eluting with 2.5% methanol in methylene chloride. mp >290°C. ¹NMR (d₆-DMSO) δ 1.18(s, 9H), 10.85 (s, 1H), 11.85 (s, 1H), 12.42 (s, 1H). Anal. Calc. for C₁₁H₁₂N₄O₂I₂: C, 2718; H 249; N, 11.53; I, 52.22. Found: C, 27.51; H, 2.51; N, 11.27;I, 52.02.

In a similar fashion but starting with 4-hydroxy-6-methylpyrrolo[2,3-d]pyrimidine and 4-hydroxypyrrolo-[2,3-d]pyrimidine (7-deazahypoxanthine) there are respectively obtained 2,3-diiodo-4-hydroxy-6-methylpyrrolo[2,3-d]pyrimidine and 2,3-diiodo-4-hydroxypyrrolo-[2,3-d]pyrimidine, mp >205°C (compound loses iodine). ¹NMR (d₆-DMSO) δ 7.79 (s, 1H), 11.93 (s, 1H), 12.74 (s, 1H).

To a mixture of 4.86 g of 2,3-diiodo-4-hydroxy-6-pivaloylaminopyrrolo[2,3-d]pyrimidine in 100 mL of glacial acetic acid and 25 mL of water are added 1.3 g (20 mmol) of zinc powder. The mixture is stirred at ambient temperature for 18 hours, diluted with 500 mL of water, and cooled. The solid is collected through filtration and dried under vacuum over phosphorus pentoxide to yield 3-iodo-4-hydroxy-6-pivaloylaminopyrrolo[2,3-d]pyrimidine which can be purified further by chromatography over silica eluting with 2.5% methanol in methylene chloride. mp >240°C. ¹NMR (d₆-DMSO) δ 1.20(s, 9H), 7.12 (d, J = 1.8 Hz, 1 H), 10.82 (s, 1H), 11.79 (s, 1H), 11.89 (s, 1H). Anal. Calc. for C₁₁H₁₃N₄O₂I: C, 36.69; H 3.64; N, 15.56; I, 35.24. Found: C, 36.91; H, 3.58; N, 15.65; I, 35.56.

In a similar fashion from 2,3-diiodo-4-hydroxy-6-methylpyrrolo[2,3-d]pyrimidine and 2,3-diiodo-4-hydroxypyrrolo[2,3-d]pyrimidine, there are respectively obtained 3-iodo-4-hydroxy-6-methylpyrrolo[2,3-d]pyrimidine and 3-iodo-4-hydroxypyrrolo[2,3-d]pyrimidine, mp >245°C (compound loses iodine). ¹NMR (d₆-DMSO) δ 7.20 (d, J = 2.2 Hz, 1H), 7.82 (d, J = 2.8 Hz, 1H), 11.85 (d, J = 1.1 Hz, 1H), 12.17 (s, 1H).

### Example 2

### DIMETHYL N-[4-(4-HYDROXY-6-PIVALOYLAMINO-PYRROLO[2,3-d]PYRIMIDIN-3-YLETHYNYL)BENZOYL]-L-GLUTAMATE

To a mixture of 3.6 g (10 mmol) of well-dried 3-iodo-4-hydroxy-6-pivaloylaminopyrrolo[2,3-d]pyrimidine in 40 mL of dimethylformamide are added 4.0 g (13.19 mmol) of dimethyl N-(4-ethynylbenzoyl)-L-glutamate, 0.38 g of copper (I) iodide, 3 mL of triethylamine, and 1.0 g of tetrakis-(triphenylphosphine)palladium. This mixture is stirred at ambient temperatures for two hours and then poured into 500 mL of water. The solid is collected by filtration, air dried, and then refluxed in 200 mL of methanol. The mixture is cooled and the solid collected by filtration, dissolved in two liters of 10% methanol in methylene chloride, and chromatographed over silica. Initial black bands are rechromatographed and the combined colorless bands from the first and second runs are evaporated to give 3.5 g of dimethyl N-[4-(4-hydroxy-6-pivaloylaminopyrrolo[2,3-d]pyrimidin-3-ylethynyl)benzoyl]-L-glutamate which can be purified further by recrystallization from 50% methanol in methylene chloride. mp 280-285°C. ¹NMR (d₆-DMSO) δ 1.21 (s, 9H), 1.96-2.15 (m, 2H), 2.44 (t, J = 7.5 Hz, 2H) 3.56 (s, 3H), 3.62 (s, 3H), 4.40-4.45 (m, 1H), 7.43 (s, 1H), 7.53 (d, J = 8.4 Hz, 2 H), 7.87 (d, J = 8.4 Hz, 2 H), 8.82 (d, J = 7.4 Hz, 1 H), 10.95 (s, 1H), 11.95 (s, 1H). Anal. Calc. for C₂₇H₂₉N₅O₇: C, 60.56; H 5.46; N, 13.08. Found: C, 60.55; H, 5.46; N, 12.89.

In as similar fashion by substituting an equivalent amount of dimethyl N-(pent-4-ynoyl)-L-glutamate, dimethyl N-(hept-6-enoyl)-L-glutamate, and dimethyl N-(hex-5-ynoyl)-L-glutamate for dimethyl N-(4-ethynylbenzoyl)glutamate in the foregoing procedure, there are obtained dimethyl N-[5-(4-hydroxy-6-pivaloylaminopyrrolo[2,3-d]pyrimidin-3-yl)pent-4-ynoyl]-L-glutamate, dimethyl N-[7-(4-hydroxy-6-pivaloylaminopyrrolo[2,3-d]pyrimidin-3-yl)hept-6-enoyl]-L-glutamate, and dimethyl N-[6-(4-hydroxy-6-pivaloylaminopyrrolo[2,3-d]pyrimidin-3-yl)hex-5-ynoyl]-L-glutamate.

Dimethyl N-(hex-5-ynoyl)-L-glutamate can be obtained in the manner described generally in U.S. Patent No. 4,882,334 issued November 21, 1989, the disclosure of which is incorporated herein by reference, by allowing hex-5-ynoic acid chloride (obtained by treating hex-5-ynoic acid with thionyl chloride) to react with dimethyl L-glutamate in the presence of an acid acceptor such as triethylamine. Hex-5-ynoic acid in turn can be prepared, for example, by alkaline hydrolysis of 5-cyanopent-1-yne.

### Example 3

### DIMETHYL N-[5-(4-HYDROXY-6-PIVALOYLAMINO-PYRROLO[2,3-d]PYRIMIDIN-3-YL}ETHYNYL)THIEN-2-YLCARBONYL]-L-GLUTAMATE

A mixture of 2.0 g of 3-iodo-4-hydroxy-6-pivaloylaminopyrrolo[2,3-d]pyrimidine, 1.2 g. of trimethylsilylacetylene, 0.1 g of palladium chloride, 0.23 g of triphenylphosphine, 0.06 g of cuprous iodide, and 2.6 g of triethylamine in 100 mL of acetonitrile is heated in a sealed tube for 1.5 hours at 50°C and then at reflux for 3 hours. The solvent is removed under reduced pressure and the residue triturated with 1:1 ethyl acetate:hexanes and filtered. The solid thus collected is dissolved in methylene chloride and this solution is passed through a pad of silica gel eluting with 1% methanol on methylene chloride. The eluate is concentrated to yield 3-trimethylsilylethynyl-4-hydroxy-6-pivaloylaminopyrrolo[2,3-d]pyrimidine.

To a solution of 1.5 g of 3-trimethylsilylethynyl-4-hydroxy-6-pivaloylaminopyrrolo[2,3-d]pyrimidine in 100 mL of anhydrous tetrahydrofuran cooled to 0°C are added under nitrogen 4.75 mL of 1M tetrabutylammonium fluoride in anhydrous tetrahydrofuran. After 5 minutes, the reaction mixture is allowed to attain room temperature and is then stirred for 2 hours. The solvent is removed under reduced pressure and the residue purified by chromatography over silica gel to yield 3-ethynyl-4-hydroxy-6-pivaloylaminopyrrolo[2,3-d]pyrimidine.

A mixture of 1.70 g. of 3-ethynyl-4-hydroxy-6-pivaloylaminopyrrolo[2,3-d]pyrimidine, 2.30 g. of dimethyl N-(5-bromothien-2-ylcarbonyl)-L-glutamate (prepared as described in U.S. Patent No. 4,882,334 issued November 21, 1989, the disclosure of which is incorporated herein by reference), 44 mg. of palladium chloride, 130 mg. of triphenylphosphine, 25 mg. of cuprous iodide, and 1.13 mL. of triethylamine in 30 mL. of acetonitrile is heated at reflux for 3 hours and then cooled to ambient temperature. The solvent is removed under reduced pressure and the residue column chromatographed (Waters 500) eluting with 1:19 methanol:methylene chloride to yield dimethyl N-[5-(4-hydroxy-6-pivaloylaminopyrrolo[2,3-d]pyrimidin-3-yl}ethynyl)thien-2-ylcarbonyl]-L-glutamate.

By substituting equivalent amounts of diethyl N-(4-bromothien-2-ylcarbony)-L-glutamate, and diethyl N-(5-bromothien-3-ylcarbony)-L-glutamate in the foregoing procedure, there are respectively obtained diethyl N-[4-(4-hydroxy-6-pivaloylaminopyrrolo[2,3-d]pyrimidin-3-ylethynyl)thien-2-ylcarbonyl]-L-glutamate and diethyl N-[5-(4-hydroxy-6-pivaloylaminopyrrolo[2,3-d]pyrimidin-3-ylethynyl)thien-3-ylcarbonyl]-L-glutamate.

Similarly from dimethyl N-(2-fluoro-4-iodobenzoyl)-L-glutamate and dimethyl N-(3-fluoro-4-iodobenzoyl)-L-glutamate (prepared as described in U.S. Patent No. 4,882,334 issued November 21, 1989, the disclosure of which is incorporated herein by reference), there are respectively obtained dimethyl N-[2-fluoro-4-(4-hydroxy-6-pivaloylaminopyrrolo[2,3-d]pyrimidin-3-yl-ethynyl)benzoyl]-L-glutamate and dimethyl N-[3-fluoro-4-(4-hydroxy-6-pivaloylaminopyrrolo[2,3-d]pyrimidin-3-ylethynyl)benzoyl]-L-glutamate.

### Example 4

### DIMETHYL N-[4-(4-HYDROXYPYRROLO[2,3-d]PYRIMIDIN-3-YL-ETHYNYL)BENZOYL]-L-GLUTAMATE

By allowing 3-iodo-4-hydroxypyrrolo[2,3-d]pyrimidine to react with dimethyl N-(4-ethynylbenzoyl)-L-glutamate in the manner described in Example 2, there is respectively obtained dimethyl N-[4-(4-hydroxy-pyrrolo[2,3-d]pyrimidin-3-ylethynyl)benzoyl]-L-glutamate which is purified by chromatography over silica, mp 160°C (dec.). ¹NMR (d₆-DMSO) δ 1.98-2.15 (m, 2H), 2.45 (t, J = 7.5 Hz, 2H) 3.57 (s, 3H), 3.64 (s, 3H), 4.40-4.45 (m, 1H), 7.51 ((d, J = 2.5 Hz, 1H), 7.55 (d, J = 8.2 Hz, 2 H), 7.87 (s, 1H), 7.90 (d, J = 8.2 Hz, 1 H), 11.97 ((d, J = 3.7 Hz, 1 H), 12.31 (s, 1H).

Alternatively, by substituting equivalent amounts of methyl 4-ethynylbenzoate, 4-ethynyltoluene, 4-ethynylbenzene, 4-ethynylchlorobenzene, 4-ethynylflourobenzene, 3-ethynylflourobenzene, and 1-methoxy-4-ethynylbenzene in the procedure of Example 2, there are obtained methyl 4-(4-hydroxy-6-pivaloylaminopyrrolo-[2,3-d]pyrimidin-3-ylethynyl)benzoate, 3-(4-methylphenyl)ethynyl-4-hydroxy-6-pivaloylaminopyrrolo[2,3-d]-pyrimidine, 3-phenylethynyl-4-hydroxy-6-pivaloylamino-pyrrolo[2,3-d]pyrimidine, 3-(4-chlorophenyl)ethynyl-4-hydroxy-6-pivaloylaminopyrrolo[2,3-d]pyrimidine, 3-(4-fluorophenyl)ethynyl-4-hydroxy-6-pivaloylaminopyrrolo-[2,3-d]pyrimidine, 3-(3-flourophenyl)ethynyl-4-hydroxy-6-pivaloyl-aminopyrrolo[2,3-d]pyrimidine, and 3-(4-methoxyphenyl)ethynyl-4-hydroxy-6-pivaloylaminopyrrolo[2,3-d]pyrimidine.

Use of 3-iodo-4-hydroxypyrrolo[2,3-d]pyrimidine in place of 3-iodo-4-hydroxy-6-pivaloylaminopyrrolo[2,3-d]pyrimidine with methyl 4-ethynylbenzoate, 4-ethynyl-toluene, 4-ethynylchlorobenzene, 4-ethynylfluorobenzene, 3-ethynylfluorobenzene, and 1-methoxy-4-ethynylbenzene yields respectively methyl 4-(4-hydroxypyrrolo-[2,3-d]pyrimidin-3-ylethynyl)benzoate, 3-(4-methylphenyl)ethynyl-4-hydroxypyrrolo[2,3-d]pyrimidine, 3-phenylethynyl-4-hydroxypyrrolo[2,3-d]pyrimidine, 3-(4-chlorophenyl)ethynyl-4-hydroxypyrrolo[2,3-d]pyrimidine, 3-(4-fluorophenyl]ethynyl-4-hydroxypyrrolo[2,3-d]pyrimidine, 3-(4-methoxyphenyl)ethynyl-4-hydroxypyrrolo[2,3-d]pyrimidine.

Ten grams of 3-iodo-4-hydroxy-6-methylpyrrolo[2,3-d]pyrimidine are allowed to react with 2.19 g of 80% sodium hydride oil dispersion and 75 ml of dimethylformamide with the exclusion of moisture. After 30 minutes, 6.02 g of chlormethyl pivalate are added. This mixture is stirred for three hours poured into water, and neutralized with acetic acid. The solid is chromatograohed on silica gel with acetone-dichloromethane to yield 3-iodo-4-hydroxy-1,5-bis(pivaolyloxy)-6-methylpyrrolo[2,3-d]pyrimidine, m.p. 155°C initially, followed by 3-iodo-4-hydroxy-5-pivaloyloxy-6-methylpyrrolo[2,3-d]pyrimidine, m.p. 236°C.

Use of 3-iodo-4-hydroxy-5-pivaloyloxy-6-methylpyrrolo[2,3-d]pyrimidine in the procedure of Example 2 then yields dimethyl N-[4-(4-hydroxy-5-pivaloyloxy-6-methylpyrrolo[2,3-d]pyrimidin-3-ylethynyl)benzoyl]-L-glutamate, m.p. 196°C Anal. Calc. for C29H32N4O8: C, 61.70; H, 5.71; N, 9.92. Found: C, 61.90; H, 5.71; N, 9.95.

Use of 3-iodo-4-hydroxy-5-pivaloyloxy-6-methylpyrrolo[2,3-d]pyrimidine in place of 3-iodo-4-hydroxy-6-pivaloylaminopyrrolo[2,3-d]pyrimidine with methyl 4-ethynylbenzoate, 4-ethynyltoluene, 4-ethynylbenzene, 3-ethynylfluorobenzene, and 1-methoxy-4-ethynylbenzene yields methyl 4-(4-hydroxy-5-pivaloyloxy-6-methyl-pyrrolo[2,3-d]pyrimidin-3-ylethynyl)benzoate, 3-(4-methylphenyl)-ethynyl-4-hydroxy-5-pivaloyloxy-6-methyl-pyrrolo[2,3-d]pyrimidine, 3-phenylethynyl-4-hydroxy-5-pivaloyloxy-6-methylpyrrolo[2,3-d]pyrimidine, 3-(4-chlorophenyl)-ethynyl-4-hydroxy-5-pivaloyloxy-6-methyl-pyrrolo[2,3-d]pyrimidine, 3-(4-fluorophenyl)-ethynyl-4-hydroxy-5-pivaloyloxy-6-methylpyrrolo[2,3-d]pyrimidine, and 3-(4-methoxyphenyl)-ethynyl-4-hydroxy-5-pivaloyloxy-6-methylpyrrolo[2,3-d]pyrimidine.

### Example 5

### DIMETHYL N-{4-[2-(4-HYDROXY-6-PIVALOYLAMINO-PYRROLO-[2,3-d]PYRIMIDIN-3-YL)ETHYL]BENZOYL}-L-GLUTAMATE

A mixture of 1.0 g of dimethyl N-[4-(4-hydroxy-6-pivaloylaminopyrrolo[2,3-d]pyrimidin-3-ylethynyl)benzoyl]-L-glutamate in 250 mL of 50% methanol in methylene chloride and 0.8 g of 3% palladium-on-carbon is hydrogenated at 50 p.s.i. for three hours, filtered, and concentrated under reduced pressure. The solid is collected by filtration and dried to yield 0.72 g of dimethyl N-{4-[2-(4-hydroxy-6-pivaloylaminopyrrolo[2,3-d]pyrimidin-3-yl)ethyl]benzoyl}-L-glutamate. mp 247°C. ¹NMR (d₆-DMSO) δ 1.21 (s, 9H), 1.90-2.12 (m, 2H), 2.42 (t, J = 7.4 Hz, 2H), 2.92 (t, J = 4 Hz, 2H), 2.97 (t, J = 4 Hz, 2H), 3.55 (s, 3H), 3.61 (s, 3H), 4.38-4.45 (m, 1H), 6.61 (s 1H), 7.27 (d, J = 8.2 Hz, 2 H), 7.75 (d, J = 8.2 Hz, 2 H), 8.64 (d, J = 7.4 Hz, 1 H), 10.75 (s, 1H), 11.22 (s, 1H). Anal. Calc. for C₂₇H₃₃N₅O₇: C, 60.10; H 6.17; N, 12.98. Found: C, 59.94; H, 6.15; N, 12.72.

### Example 6

### DIMETHYL N-{5-[2-(4-HYDROXY-6-PIVALOYLAMINOPYRROLO-[2,3-d]PYRIMIDIN-3-YL)ETHYL]THIEN-2-YLCARBONYL}-L-GLUTAMATE

By subjecting dimethyl N-[5-(4-hydroxy-6-pivaloylaminopyrrolo[2,3-d]pyrimidin-3-yl}ethynyl)thien-2-ylcarbonyl]-L-glutamate to the hydrogenation procedure of Example 5, there is obtained dimethyl N-{5-[2-(4-hydroxy-6-pivaloylaminopyrrolo[2,3-d]pyrimidin-3-yl)ethyl]thien-2-ylcarbonyl}-L-glutamate.

Similarly the following compounds are subjected to the hydrogenation of Example 5:
(a) dimethyl N-[2-fluoro-4-(4-hydroxy-6-pivaloylamino-pyrrolo[2,3-d]pyrimidin-3-ylethynyl)benzoyl]-L-glutamate;
(b) dimethyl N-[3-fluoro-4-(4-hydroxy-6-pivaloylamino-pyrrolo[2,3-d]pyrimidin-3-ylethynyl)benzoyl]-L-glutamate;
(c) diethyl N-[4-(4-hydroxy-6-pivaloylaminopyrrolo-[2,3-d]pyrimidin-3-ylethynyl)thien-2-ylcarbonyl]-L-glutamate;
(d) diethyl N-[5-(4-hydroxy-6-pivaloylaminopyrrolo-[2,3-d]pyrimidin-3-ylethynyl)thien-3-ylcarbonyl]-L-glutamate;
(e) dimethyl N-[5-(4-hydroxy-6-pivaloylaminopyr-rolo[2,3-d]pyrimidin-3-yl)pent-4-ynoyl]-L-glutamate;
(f) dimethyl N-[7-(4-hydroxy-6-pivaloylaminopyrrolo-[2,3-d]pyrimidin-3-yl)hept-6-enoyl]-L-glutamate;
(g) dimethyl N-[6-(4-hydroxy-6-pivaloylaminopyrrolo-[2,3-d]pyrimidin-3-yl)hex-5-ynoyl]-L-glutamate;
(h) methyl 4-(4-hydroxy-6-pivaloylaminopyrrolo[2,3-d]pyrimidin-3-ylethynyl)benzoate;
(i) 3-(4-methylphenyl)ethynyl-4-hydroxy-6-pival-oylaminopyrrolo[2,3-d]pyrimidine;
(j) 3-phenylethynyl-4-hydroxy-6-pivaloylaminopyrrolo[2,3-d]pyrimidine;
(k) 3-(4-chlorophenyl)ethynyl-4-hydroxy-6-pivaloylaminopyrrolo[2,3-d]pyrimidine;
(l) 3-(4-fluorophenyl)ethynyl-4-hydroxy-6-pivaloylaminopyrrolo[2,3-d]pyrimidine;
(m) 3-(3-fluorophenyl)ethynyl-4-hydroxy-6-pivaloylaminopyrrolo[2,3-d]pyrimidine;
(n) 3-(4-methoxyphenyl)ethynyl-4-hydroxy-6-pivaloylaminopyrrolo[2,3-d]pyrimidine;
(o) methyl 4-(4-hydroxy-5-pivaloyloxy-6-methylpyrrolo[2,3d]pyrimidin-3-ylethynyl)benzoate;
(p) 3-(4-methylphenyl)ethynyl-4-hydroxy-5-pivaloyloxy6-methylpyrrolo[2,3-d]pyrimidine;
(q) 3-phenylethynyl-4-hydroxy-5-pivaloyloxy-6-methylpyrrolo[2,3-d]pyrimidine;
(r) 3-(4-chlorophenyl)ethynyl-4-hydroxy-5-pivaloyloxy-6-methylpyrrolo[2,3-d]pyrimidine;
(s) 3-(4-fluorophenyl)ethynyl-4-hydroxy-5-pivaloyloxy6-methylpyrrolo[2,3-d]pyrimidine;
(t) 3-(4-methoxyphenyl)ethynyl-4-hydroxy-5-pivaloyloxy-6-methylpyrrolo[2,3-d]pyrimidine;
(u) methyl 4-(4-hydroxypyrrolo[2,3-d]pyrimidin-3-ylethynyl)benzoate;
(v) 3-(4-methylphenyl)ethynyl-4-hydroxypyrrolo[2,3-d]pyrimidine;
(w) 3-phenylethynyl-4-hydroxypyrrolo[2,3-d]pyrimidine;
(x) 3-(4-chlorophenyl)ethynyl-4-hydroxypyrrolo[2,3-d]pyrimidine;
(y) 3-(4-fluorophenyl)ethynyl-4-hydroxypyrrolo[2,3-d]pyrimidine; and
(z) 3-(4-methoxyphenyl)ethynyl-4-hydroxypyrrolo[2,3-d]pyrimidine.

There are respectively obtained:
(a) dimethyl N-[2-fluoro-4-(4-hydroxy-6-pivaloylamino-pyrrolo[2,3-d]pyrimidin-3-ylethyl)benzoyl]-L-glutamate;
(b) dimethyl N-[3-fluoro-4-(4-hydroxy-6-pivaloylamino-pyrrolo[2,3-d]pyrimidin-3-ylethyl)benzoyl]-L-glutamate;
(c) diethyl N-[4-(4-hydroxy-6-pivaloylaminopyrrolo-[2,3-d]pyrimidin-3-ylethyl)thien-2-ylcarbonyl]-L-glutamate;
(d) diethyl N-[5-(4-hydroxy-6-pivaloylaminopyrrolo-[2,3-d]pyrimidin-3-ylethyl)thien-3-ylcarbonyl]-L-glutamate;
(e) dimethyl N-[5-(4-hydroxy-6-pivaloylaminopyr-rolo[2,3-d]pyrimidin-3-yl)pentyl]-L-glutamate;
(f) dimethyl N-[7-(4-hydroxy-6-pivaloylaminopyrrolo-[2,3-d]pyrimidin-3-yl)heptyl]-L-glutamate;
(g) dimethyl N-[6-(4-hydroxy-6-pivaloylaminopyrrolo[2,3-d]pyrimidin-3-yl)hexyl]-L-glutamate;
(h) methyl 4-[2-(4-hydroxy-6-pivaloylaminopyrrolo[2,3-d]pyrimidin-3-yl)ethyl]benzoate;
(i) 3-[2-(4-methylphenyl)ethyl]-4-hydroxy-6-pivaloylaminopyrrolo[2,3-d]pyrimidine;
(j) 3-(2-phenylethyl)-4-hydroxy-6-pivaloylaminopyrrolo[2,3-d]pyrimidine;
(k) 3-[2-(4-chlorophenyl)ethyl]-4-hydroxy-6-pivaloylaminopyrrolo[2,3-d]pyrimidine;
(1) 3-[2-(4-fluorophenyl)ethyl]-4-hydroxy-6-pivaloylaminopyrrolo[2,3-d]pyrimidine;
(m) 3-[2-(3-flourophenyl)ethyl]-4-hydroxy-6-pivaloylaminopyrrolo[2,3-d]pyrimidine;
(n) 3-[2-(4-methoxyphenyl)ethyl]-4-hydroxy-6-pivaloylaminopyrrolo[2,3-d]pyrimidine;
(o) methyl 4-[2-(4-hydroxy-6-methylpyrrolo[2,3-d]pyrimidin-3-yl)ethyl]benzoate;
(p) 3-[2-(4-methylphenyl)ethyl]-4-hydroxy-5-pivaloyloxy-6-methylpyrrolo[2,3-d]pyrimidine;
(q) 3-[2-(4-chlorophenyl)ethyl]-4-hydroxy-5-pivaloyloxy-6-methylpyrrolo[2,3-d]pyrimidine;
(r) 3-[2-(4-fluorophenyl)ethyl]-4-hydroxy-5-pivaloyloxy-6-methylpyrrolo[2,3-d]pyrimidine;
(s) 3-[2-(4-methoxyphenyl)ethyl]-4-hydroxy-5-pivaloyloxy-6-methylpyrrolo[2,3-d]pyrimidine;
(t) methyl 4-[2-(4-hydroxypyrrolo[2,3-d]pyrimidin-3-yl)ethyl]benzoate;
(u) 3-[2-(4-methylphenyl)ethyl]-4-hydroxypyrrolo[2,3-d]pyrimidine;
(v) 3-(2-phenylethyl)-4-hydroxypyrrolo[2,3-d]pyrimidine;
(w) 3-[2-(4-chlorophenyl)ethyl]-4-hydroxypyrrolo[2,3-d]pyrimidine;
(x) 3-[2-(4-fluorophenyl)ethyl]-4-hydroxypyrrolo[2,3-d]pyrimidine; and
(y) 3-[2-(4-methoxyphenyl)ethyl]-4-hydroxypyrrolo[2,3-d]pyrimidine.
(z) 3-[2-(4-methoxyphenyl)ethyl]-4-hydroxypyrrolo[2,3-d]pyrimidine.

### Example 7

### DIMETHYL N-{4-[2-(4-HYDROXYPYRROLO[2,3-d]PYRIMIDIN-3-YL)ETHYL]BENZOYL}-L-GLUTAMATE

A mixture of 1.1 g of dimethyl N-[4-(4-hydroxy-pyrrolo[2,3-d]pyrimidin-3-ylethynyl)benzoyl]-L-glutamate in 100 mL of 50% methanol in methylene chloride and 0.8 g of 3% palladium-on-carbon is hydrogenated at 50 p.s.i. for 24 hours, filtered, and concentrated under reduced pressure. Ether is added to the residue and the solid is collected by filtration and dried to yield 0.67 g of dimethyl N-{4-[2-(4-hydroxyamino-pyrrolo[2,3-d]pyrimidin-3-yl)ethyl]benzoyl}-L-glutamate. mp 170-172°C. ¹NMR (d₆-DMSO) δ 1.94-2.14 (m, 2H), 2.44 (t, J = 7.4 Hz, 2H), 2.93-3.02 (m, 2H), 3.57 (s, 3H), 3.63 (s, 3H), 4.40-4.70 (m, 1H), 6.71 (s, 1H), 7.29 (d, J = 8.2 Hz, 2 H), 7.77 (m, 3 H), 8.66 (d, J = 7.4 Hz, 1 H), 11.52 (s, 1H), 11.71 (s, 1H).

In a similar fashion from dimethyl N-[4-(4-hydroxy-5-pivaloyloxy-6-methylpyrrolo[2,3-d]pyrimidin-3-ylethynyl)benzoyl]-L-glutamate, there is obtained according to this procedure dimethyl N-{4-[2-(4-hydroxy-5-pivaloyloxy-6-methylpyrrolo[2,3-d]pyrimidin-3-yl)ethyl]benzoyl}-L-glutamate.

### Example 8

### N-{4-[2-(4-HYDROXY-6-PIVALOYLAMINOPYRROLO[2,3-d]-PYRIMIDIN-3-YL)ETHYL]BENZOYL}-L-GLUTAMIC ACID

A mixture of 1.5 g of dimethyl N-{4-[2-(4-hydroxy-6-pivaloylaminopyrrolo[2,3-d]pyrimidin-3-yl)ethyl]benzoyl)-L-glutamate in 10 mL of 1N sodium hydroxide is stirred at ambient temperatures for three days to form the sodium salt of N-{4-[2-(4-hydroxy-6-aminopyrrolo-[2,3-d]pyrimidin-3-yl)ethyl]benzoyl}-L-glutamic acid. This is neutralized with glacial acetic acid. The solid which forms is collected by filtration and recrystallized from 50% methanol in methylene chloride to give 0.8 g (67%) of N-{4-[2-(4-hydroxy-6-amino-pyrrolo[2,3-d]pyrimidin-3-yl)ethyl]benzoyl}-L-glutamic acid. ¹NMR (d₆-DMSO) δ 1.80-2.00 (m, 2H), 2.10-2.30 (m, 2H), 2.77-2.820 (m, 2H), 2.89-2.93 (m, 2H), 4.13-4.19 (m, 2H), 6.25 (d, J = 1.3 Hz, 1H), 7.23 (d, J = 8.1 Hz, 2 H), 7.69 (d, J = 8.1 Hz, 2 H), 8.13 (d, J = 6.7 Hz, 1 H), 10.55 (s, 1H).

### Example 9

### N-[4-{1-HYDROXY-3-(4-HYDROXY-6-AMINOPYRROLO[2,3-d]-PYRIMIDIN-3-YL)PROP-2-YL}BENZOYL]GLUTAMIC ACID

A solution of 0.3 g of diethyl N-[4-{1-hydroxy-3-(4-hydroxy-6-pivaloylaminopyrrolo[2,3-d]pyrimidin-3-yl)prop-2-yl}benzoyl]glutamate in 9 ml of 1N aqueous sodium hydroxide is stirred under nitrogen at ambient temperature for 72 hours. The reaction mixture is rendered slightly acidic (pH=∼4) with 1N hydrochloric acid and filtered. The solid thus collected is washed with water (5 ml) and cold ethanol (5 ml) and dried to give N-[4-{1-hydroxy-3-(4-hydroxy-6-aminopyrrolo[2,3-d]-pyrimidin-3-yl)prop-2-yl}benzoyl]glutamic acid.

Similarly from dimethyl N-{2-fluoro-4-[2-(4-hydroxy-6-pivaloylaminopyrrolo[2,3-d]pyrimidin-3-yl)-ethyl]benzoyl}-L-glutamate and dimethyl N-{3-fluoro-4-[2-(4-hydroxy-6-pivaloylaminopyrrolo[2,3-d]pyrimidin-3-yl)ethyl]benzoyl}-L-glutamate there are respectively obtained according to the foregoing procedure N-{2-fluoro-4-[2-(4-hydroxy-6-aminopyrrolo[2,3-d]pyrimidin-3-yl)ethyl]benzoyl}-L-glutamic acid, m.p. 230°C (foaming), >300°C (dec.) and N-{3-fluoro-4-[2-(4-hydroxy-6-aminopyrrolo[2,3-d]pyrimidin-3-yl)ethyl]-benzoyl}-L-glutamic acid, m.p. >300°C. (dec.).

In an analogous fashion to the foregoing procedure, there are respectively obtained from diethyl N-{4-[2-(4-hydroxy-6-pivaloylaminopyrrolo[2,3-d]pyrimidin-3-yl)ethyl]thien-2-ylcarbonyl}-L-glutamate, diethyl N-{5-[2-(4-hydroxy-6-pivaloylaminopyrrolo[2,3-d]pyrimidin-3-yl)ethyl]thien-3-ylcarbonyl}-L-glutamate, and dimethyl N-{5-[2-(4-hydroxy-6-pivaloylaminopyrrolo[2,3-d]pyrimidin-3-yl)ethyl]thien-2-ylcarbonyl}-L-glutamate, the compounds N-{4-[2-(4-hydroxy-6-aminopyrrolo[2,3-d]pyrimidin-3-yl)ethyl]thien-2-ylcarbonyl}-L-glutamic acid, N-{5-[2-(4-hydroxy-6-aminopyrrolo[2,3-d]pyrimidin-3-yl)ethyl]thien-3-ylcarbonyl}-L-glutamic acid, and N-{5-[2-(4-hydroxy-6-aminopyrrolo[2,3-d]-pyrimidin-3-yl)ethyl]thien-2-ylcarbonyl}-L-glutamic acid m.p. 241-243°C.

Similarly obtained from dimethyl N-[5-(4-hydroxy-6-pivaloylaminopyrrolo[2,3-d]pyrimidin-3-yl)pentanoyl]-L-glutamate, dimethyl N-[7-(4-hydroxy-6-pivaloylamino-pyrrolo[2,3-d]pyrimidin-3-yl)heptanoyl]-L-glutamate, and dimethyl N-[6-(4-hydroxy-6-pivaloylaminopyrrolo-[2,3-d]pyrimidin-3-yl)-hexanoyl]-L-glutamate, are, respectively, N-[5-(4-hydroxy-6-aminopyrrolo[2,3-d]-pyrimidin-3-yl)pentanoyl]-L-glutamic acid, N-[7-(4-hydroxy-6-aminopyrrolo[2,3-d]pyrimidin-3-yl)heptanoyl]-L-glutamic acid, and N-[6-(4-hydroxy-6-aminopyrrolo[2,3-d]pyrimidin-3-yl)hexanoyl]-L-glutamic acid.

### Example 10

### N-{4-[2-(4-HYDROXYPYRROLO[2 3-d]PYRIMIDIN-3-YL)ETHYL]-BENZOYL}-L-GLUTAMIC ACID

A mixture of 0.5 g of dimethyl N-{4-[2-(4-hydroxypyrrolo[2,3-d]pyrimidin-3-yl)ethyl]benzoyl}-L-glutamate in 3 mL of 1N sodium hydroxide is stirred at ambient temperatures for three days to form the sodium salt of N-{4-[2-(4-hydroxy-6-aminopyrrolo[2,3-d]pyrimi-din-3-yl)ethyl]benzoyl}-L-glutamic acid. This is neutralized with hydrochloric acid. The solid which forms is collected by filtration and recrystallized from methanol by addition of water to give 0.35 g (75%) of N-{4-[2-(4-hydroxypyrrolo[2,3-d]pyrimidin-3-yl)ethyl]benzoyl}-L-glutamic acid. ¹NMR (d₆-DMSO) δ 1.88-2,12 (m, 2H), 2.33 (t, J = 7.3 Hz, 2H), 2.97 (m, 4H), 4.33-4.40 (m, 1H), 6.70 (d, J = 1.2 Hz, 1H), 7.28 (d, J = 7.0 Hz, 2 H), 7.76 (m, 3H), 8.50 (d, J = 7.6 Hz, 1H), 11.48 (s, 1H), 11.67 (s, 1H), 12.40 (br, 1H).

In a similar fashion from dimethyl N-{4-[2-(4-hydroxy-5-pivaloyloxy-6-methylpyrrolo[2,3-d]pyrimidin-3-yl)ethyl]benzoyl}-L-glutamate, there is obtained according to the foregoing procedure first the sodium salt of N-{4-[2-(4-hydroxy-6-methylpyrrolo[2,3-d]pyrimidin-3-yl)ethyl]benzoyl}-L-glutamic acid which upon neutralization with glacial acetic acid yields N-{4-[2-(4-hydroxy-6-methylpyrrolo[2,3-d]pyrimidin-3-yl)ethyl]-benzoyl}-L-glutamic acid.

By subjecting methyl 4-[2-(4-hydroxy-5-pivaloyloxy-6-methylaminopyrrolo[2,3-d]pyrimidin-3-yl)ethyl]-benzoate; 3-[2-(4-methylphenyl)ethyl]-4-hydroxy-6-loylamino-pyrrolo[2,3-d]pyrimidine; 3-(2-phenylethyl)-4-hydroxy-6-pivaloylaminopyrrolo[2,3-d]pyrimidine; 3-[2-(4-chlorophenyl)ethyl]-4-hydroxy-6-pivaloylamino-pyrrolo[2,3-d]pyrimidine; 3-[2-(4-fluorophenyl)ethyl]4-hydroxy-6-pivaloylaminopyrrolo[2,3-d]pyrimidine; 3-[2-(3-fluorophenyl)ethyl]-4-hydroxy-6-pivaloyl-aminopyrrolo[2,3-d]pyrimidine; 3-[2-(4-methoxyphenyl)-ethyl]-4-hydroxy-6-pivaloylaminopyrrolo[2,3-d]pyrimidine; and methyl 4-[2-(4-hydroxy-6-pivaloylmethylpyrrolo[2,3-d]pyrimidin-3-yl)ethyl]benzoate to the foregoing procedure, there are respectively obtained 4-[2-(4-hydroxy-6-methylpyrrolo[2,3-d]pyrimidin-3-yl)ethyl]benzoic acid; 3-[2-(4-methylphenyl)ethyl]-4-hydroxy-6-aminopyrrolo[2,3-d]pyrimidine; 3-(2-phenylethyl)-4-hydroxy-6-aminopyrrolo[2,3-d]pyrimidine; 3-[2-(4-chlorophenyl)ethyl]-4-hydroxy-6-aminopyrrolo[2,3-d]-pyrimidine; 3-[2-(4-fluorophenyl)ethyl]-4-hydroxy-6-aminopyrrolo[2,3-d]pyrimidine; 3-[2-(3-fluorophenyl)-ethyl]-4-hydroxy-6-aminopyrrolo[2,3-d]pyrimidine, m.p. 295-298°C; 3-[2-(4-methoxyphenyl)ethyl]-4-hydroxy-6-aminopyrrolo[2,3-d]pyrimidine, m.p. 280-284°C; and 4-[2-(4-hydroxy-6-aminopyrrolo[2,3-d]pyrimidin-3-yl)-ethyl]benzoic acid, m.p. >300°C.

### Example 11

### Comparative Study of Antimetabolic Activity

Antimetabolic studies were conducted utilizing the following compounds:

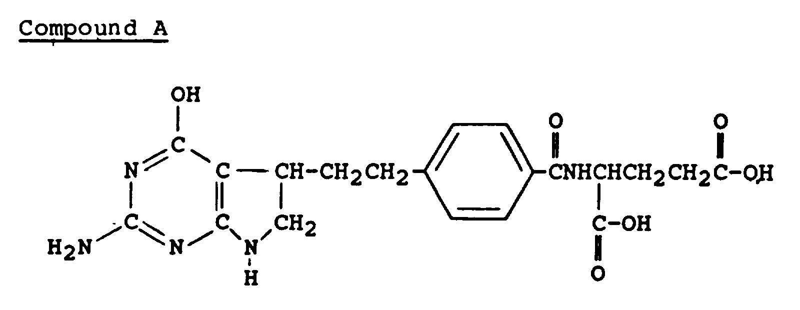

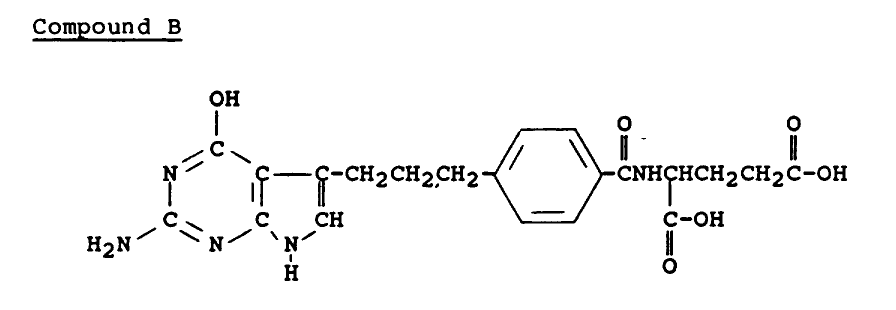

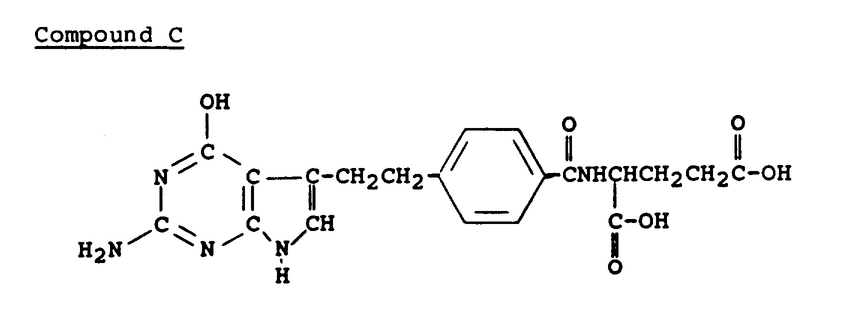

Each of the compounds was dissolved in 100% dimethylsulfoxide. Dilutions of each compound were prepared to yield a range of test concentrations as hereinafter indicated.

The inhibitory activity of the compounds were tested according to the following procedures:

### Preparation of Stock Cells

Cem human lymphoblastic leukemia cells are grown in RPMI 1640 media (MABio), supplemented with 10% dialyzed fetal bovine serum (Gibco) at 37°C in humidified atmospheric conditions of 95% air and 5% CO₂. The cells are grown in static suspension (T-flask, Corning) maintained in log phase at a concentration of 3-7 x 10⁵ cells/ml.

### Procedure

(i) 10 µl of a test compound is placed in a well (Costar 24 well clusters). This step is repeated for each test concentration of the compounds.
(ii) 500 µl of serum-free media is added to each well.
(iii) A sterile bottle containing RPMI 1640 media supplemented with 10% dialyzed fetal bovine serum, 16 mM hepes and 8 mM mops buffers and 3 x 10⁴ cells/ml is placed on a magnetic stirrer. A Wheaton peristaltic pump is used to deliver 1.5 ml of this cell suspension to each well. The resulting final volume delivered to each well is 2.0 ml at a cellular concentration of 4.8 x 10⁴ cells/well.
(iv) The cluster wells are incubated at 37°C in a humidified incubator (95% air, 5% CO₂) for 72 hours.
(v) After the incubation period, measurements of each well are taken using the ZB1 Coulter Counter particle counter and the IC₅₀ (concentration inhibiting 50% of cellular growth) is determined.
(vi) The results of this test are as follows:

**Table I**

| Concentration | Cellular Growth (%) | | |
|---|---|---|---|
| (µg/ml) | A | B | C |
| 100.0 | 13.1 | - | 14 |
| 33.3 | 15.5 | 10.8 | 12 |
| 11.1 | 16.2 | 9.6 | 12 |
| 3.7 | 15.3 | 10.4 | 13 |
| 1.23 | 16.1 | 11.3 | 14 |
| 0.411 | 21.5 | 13.0 | 13 |
| 0.137 | 63.9 | 17.7 | 14 |
| 0.045 | 100.0 | 33.5 | 14 |
| 0.015 | - | 76.9 | 25 |
| 0.005 | - | - | 96 |

Each of Compounds A, B and C exhibited in vitro inhibitory activity against human lymphoblastic leukemia cells. The relative overall in vitro inhibitory concentration (IC₅₀) of these compounds is as follows:
Compound A: 0.2 µg/ml
Compound B: 0.03 µg/ml
Compound C: 0.007 µg/ml

All compounds demonstrated inhibitory activity. When compared in vitro, Compound C was the most active of the three.

### Reversal Studies

The mechanism of action of each compound was determined through further tests employing so-called "reversal" studies. These studies supplied compounds or precursors which, alone or together, identified the specific point of enzymatic inhibition.
(a) Hypoxanthine - Hypoxanthine will reverse the inhibitory effects of a compound if the compound functions as an inhibitor of purine de novo synthesis. The availability of hypoxanthine thus allows cells to bypass purine de novo synthesis.
(b) Thymidine - Thymidine alone will reverse the inhibitory effects of a compound if the compound functions as a specific inhibitor of thymidylate synthetase.
(c) Hypoxanthine and Thymidine - If a compound is determined in (a) to not be an inhibitor of purine de novo synthesis, and in (b) to not be an inhibitor of thymidilate synthetase, then a reversal study utilizing hypoxanthine and thymidine, in combination, will determine whether the compound can produce inhibition of both pathways. Thus if this combination reverses the inhibitory effects of a compound, the compound most likely functions as an inhibitor of dihydrofolate reductase (involved in both purine and pyrimidine biosynthesis).

### Procedure

In each case, the basic procedure outlined above was followed, modified in that 250 µl of the reversal compound [or of each reversal compound in (b)] was added to the test wells, and the amount of serumless media added to each test well was decreased [or entirely eliminated in (b)] by the same amount. The reversal compounds were prepared as 8X stock solutions in phosphate buffered saline (sterile filtered through a 0.2 µ acrodisc filter - Gelman) so that 250 µl of the stock solution would yield a final concentration of 100 µM hypoxanthine and/or 5 µM thymidine.

The results of the reversal tests are as follows:
(a) Hypoxanthine (100 µM): 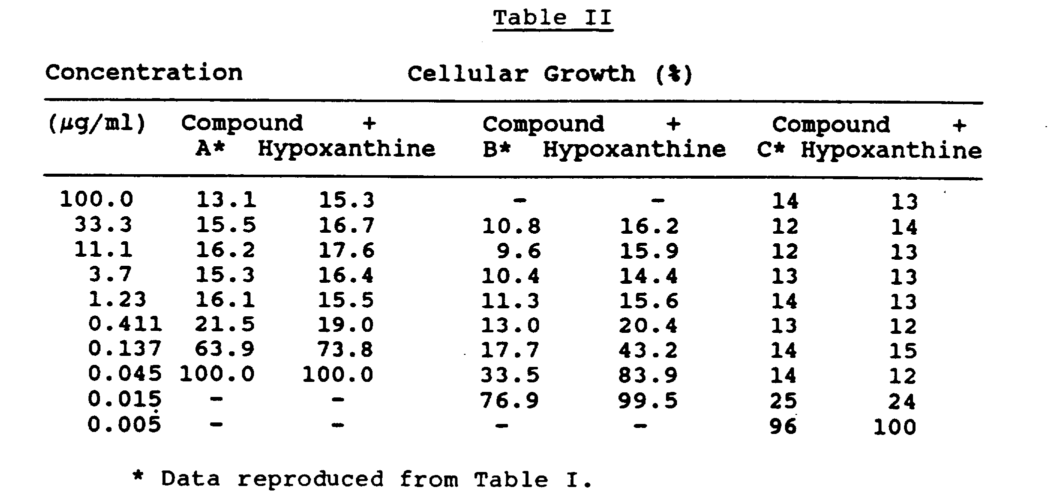
(b) Hypoxanthine (100 µM) and Thymidine (5 µM):
   Compound B was further tested to determine the secondary site of inhibitory activity. 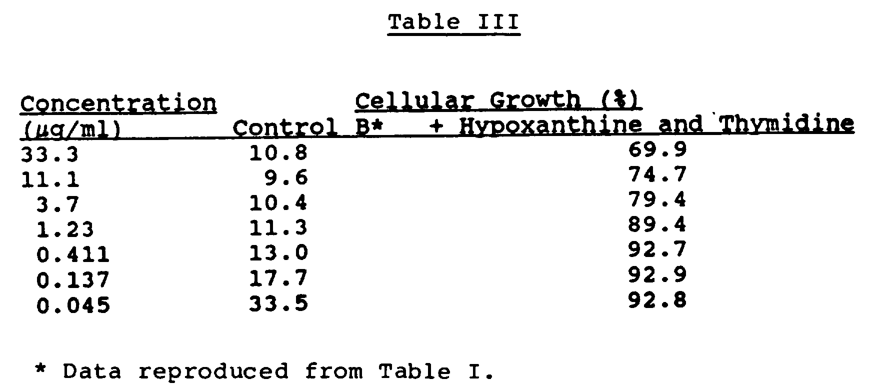
(c) Thymidine (5 µM):

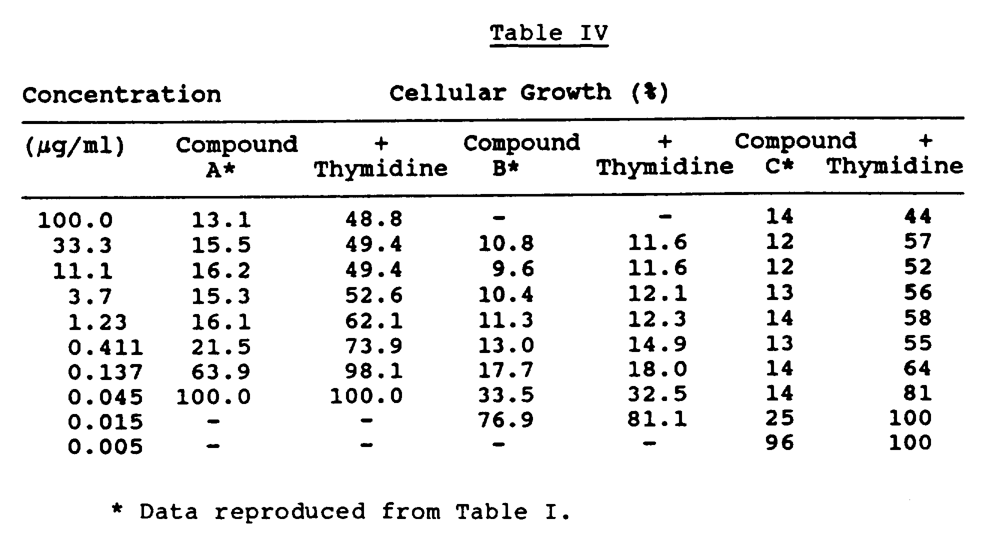

### In Vivo Activity Analysis

Compounds A and C were tested further to compare their antitumor activity in vivo.

Female mice, strain DBA/2, were inoculated with tumor cells. Treatment with the test compounds began the day following inoculation. The treatment consisted of eight daily doses i.p. (intraperitoneal) of either Compounds A and C for eight consecutive days. Tumor size was measured on the day following the last day of treatment to determine the inhibitory effect of the test compounds on tumor growth.

The results are as follows:

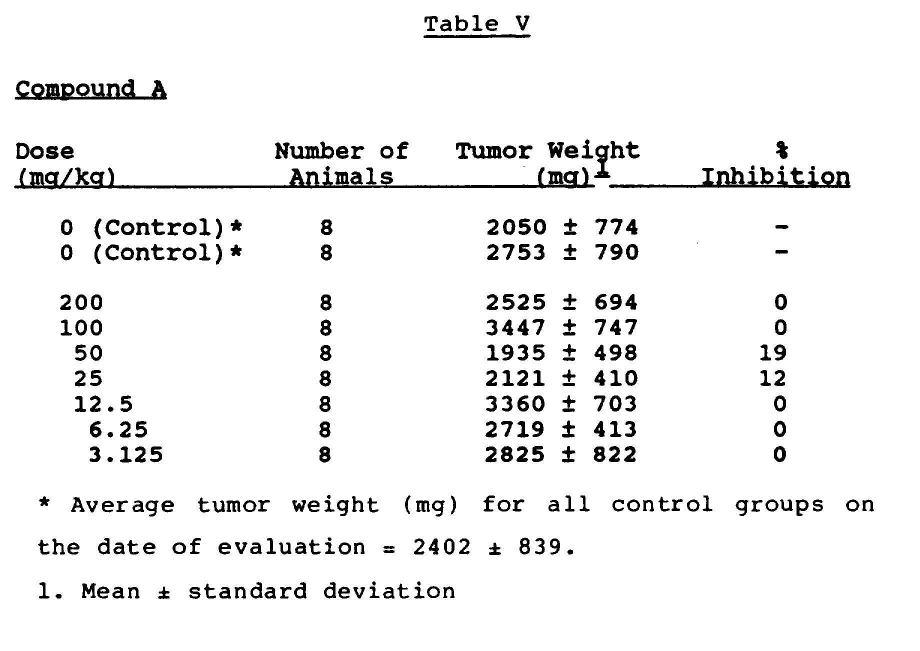

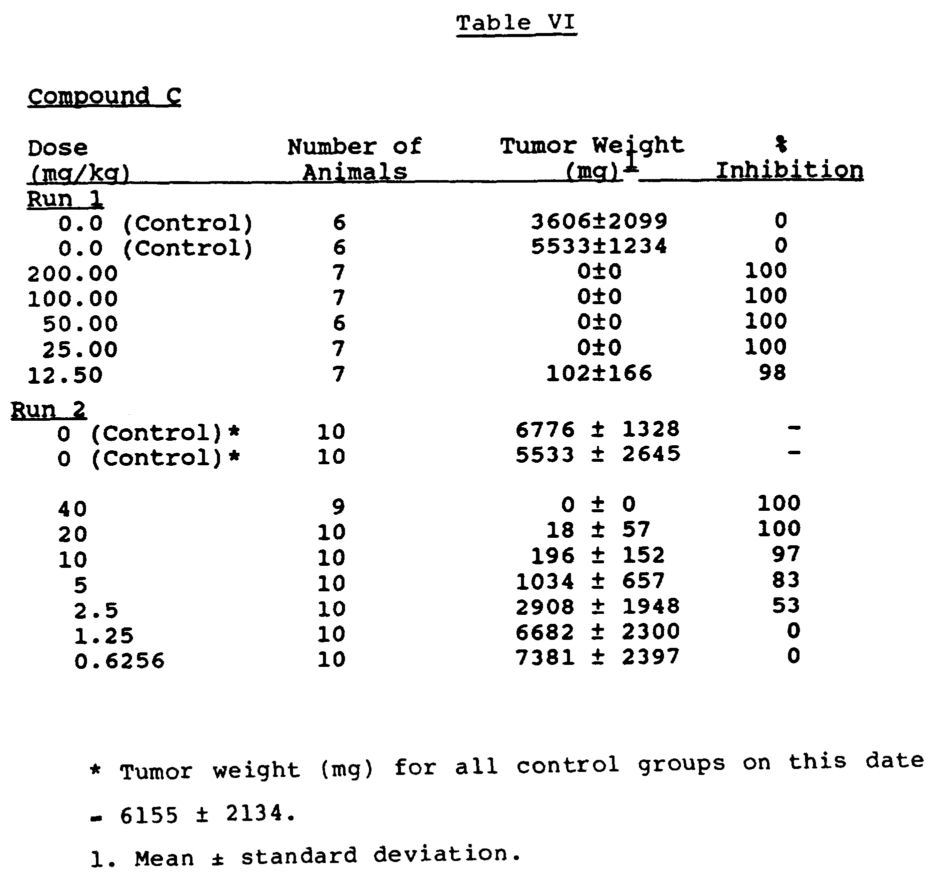

Compound A has essentially no inhibitory effect on tumor growth in vivo. While observed tumor weight was slightly lower at doses of 25 and 50 mg/kg, this is not considered to be significant since there was no reduction in tumor weight at doses of 100 or 200 mg/kg.

Compound C exhibits a marked in vivo inhibitory effect on tumor growth, with 100% inhibition at doses of as low as 20 mg/kg, and more than 50% inhibition at doses of at least 2.5 mg/kg.

Compound B acts by a different mechanism than Compounds A or C, namely inhibition of DHFR rather than TS. While in vitro, Compounds A and C appear to inhibit TS; Compound C is more active in vitro than Compound A by a factor of at least 25-fold. In vivo, Compound A is inactive as an antineoplastic agent. Compound C is highly active in vivo in combatting neoplasts.

Representative inhibition values against CCRF-CEM cell cultures for typical compounds are as follows:

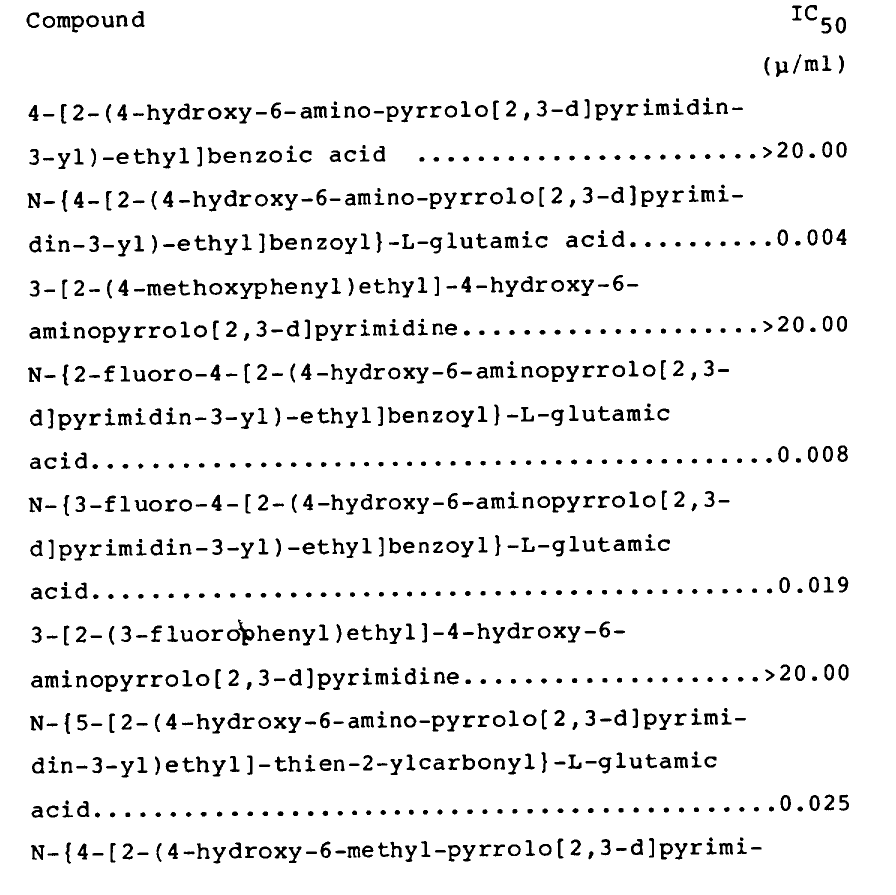

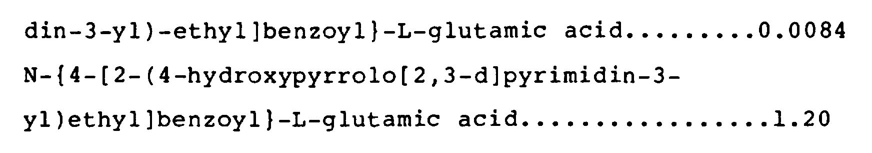

The cytotoxicity of these compounds is not reversed by the addition of hypoxanthine or AICA, suggesting that they do not inhibit the purine de novo biosynthesis pathway, but is reversed by thymidine, indicating thymidylate synthetase is the main target. Cytotoxicity is also reversed by the addition of leucovorin, indicating the cytotoxicity is due to antagonism of a folate-related mechanism.

## Claims (Claims for the following Contracting State(s): DE, GB, FR, IT, NL, SE, LI, CH, BE, AT, LU, DK)

1. A compound of the formula: 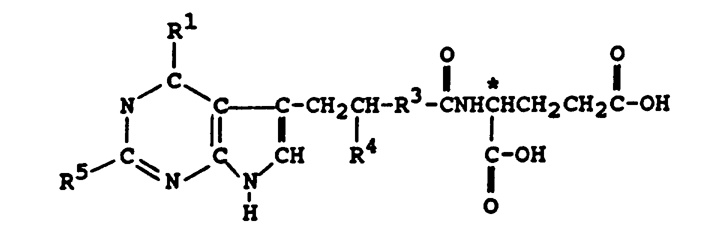 in which:
R¹ is -OH or NH₂;
R³ is 1,4-phenylene or 1,3-phenylene unsubstituted or substituted with chloro, fluoro, methyl, methoxy, or trifluoromethyl; thienediyl unsubstituted or substituted with chloro, fluoro, methyl, methoxy, or trifluoromethyl; cyclohexanediyl; or a straight or branched divalent aliphatic group of from 2 to 4 carbon atoms;
R⁴ is hydrogen; and
R⁵ is hydrogen, alkyl of 1 to 6 carbon atoms, or amino;
the configuration about the carbon atom designated * is S; and
the pharmaceutically acceptable salts thereof.

2. A compound according to claim 1 wherein R¹ is -OH; R³ is 1,4-phenylene; R⁴ is hydrogen and R⁵ is hydrogen.

3. A compound according to claim 1 wherein R¹ is -OH; R³ is 1,4-phenylene; R⁴ is hydrogen and R⁵ is methyl.

4. A compound according to claim 1 wherein R¹ is -OH; R³ is thienediyl; R⁴ is hydrogen and R⁵ is hydrogen.

5. A compound according to claim 1 wherein R¹ is -OH; R³ is thienediyl; R⁴ is hydrogen and R⁵ is methyl.

6. The compound according to claim 1 which is N-{4-[2-(4-hydroxypyrrolo[2,3-d]pyrimidin-3-yl)ethyl]benzoyl}-L-glutamic acid, and N-{4-[2-(4-hydroxy-6-methylpyrrolo[2,3-d]pyrimidin-3-yl)ethyl]benzoyl}-L-glutamic acid.

7. The compound of the formula: 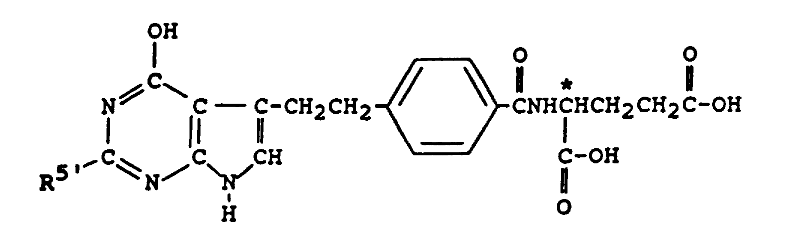 in which R^{5'} is amino or alkanoylamino of 1 to 12 carbon atoms, the configuration about the carbon atom designated * is S, and the pharmaceutically acceptable salts thereof.

8. The compound according to claim 7 which is N-{4-[2-(4-hydroxy-6-aminopyrrolo[2,3-d]pyrimidin-3-yl)ethyl]benzoyl}-L-glutamic acid and the pharmaceutically acceptable salts thereof.

9. A compound of the formula: 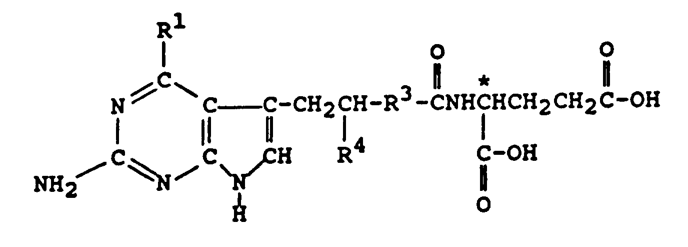 in which:
R¹ is -OH or -NH₂;
R³ is thienediyl unsubstituted or substituted with chloro, fluoro, methyl, methoxy, or trifluoromethyl; cyclohexadiyl; or a straight or branched divalent aliphatic group of from 2 to 4 carbon atoms;
R⁴ is hydrogen; and
the configuration about the carbon atom designated * is S; and
the pharmaceutically acceptable salts thereof.

10. A compound according to claim 9 wherein R¹ is -OH; R³ is thienediyl, and R⁴ is hydrogen.

11. The compound according to claim 9 which is N-{5-[2-(4-hydroxy-6-aminopyrrolo[2,3-d]pyrimidin-3-yl)ethyl]thien-2-ylcarbonyl}-L-glutamic acid; N-{4-[2-(4-hydroxy-6-aminopyrrolo[2,3-d]pyrimidin-3-yl)-ethyl]thien-2-ylcarbonyl}-L-glutamic acid; or N-{5-[2-(4-hydroxy-6-aminopyrrolo[2,3-d]pyrimidin-3-yl)ethyl]thien-3-ylcarbonyl}-L-glutamic acid.

12. A process for the preparation of compounds of the formula 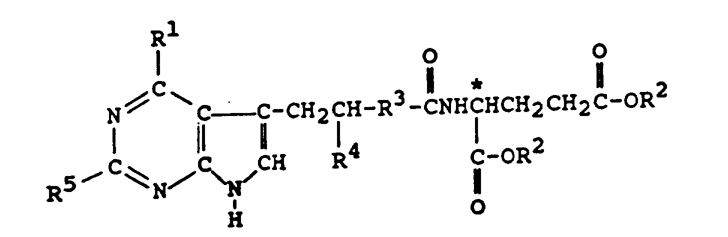 in which:
R¹ is -OH or -NH₂;
R is hydrogen or a pharmaceutically acceptable cation;
R³ is 1,4-phenylene or 1,3-phenylene unsubstituted or substituted with chloro, fluoro, methyl, methoxy, or trifluoromethyl; thienediyl unsubstituted or substituted with chloro, fluoro, methyl, methoxy, or trifluoromethyl; cyclohexanediyl; or a straight or branched divalent aliphatic group of from 2 to 4 carbon atoms;
R⁴ is hydrogen;
R⁵ is hydrogen, alkyl of 1 to 6 carbon atoms, or amino; and
the configuration about the carbon atom designated * is S;
which comprises catalytically hydrogenating a compound of the formula 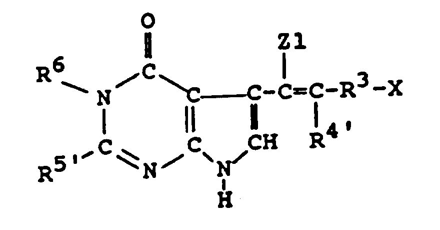 in which:
X is COOR^{2'}; or 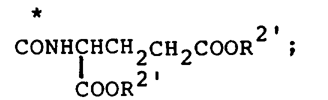
Z¹ is hydrogen, or Z¹ taken together with R^{4'} is a carbon-carbon bond;
R^{2'} is hydrogen or a carboxy protecting group;
R³ is as defined above;
R^{4'}, when taken independently of Z¹, is hydrogen;
R^{5'} is hydrogen, alkyl of 1 to 6 carbon atoms, amino, or an amino protecting group; and
R⁶ is hydrogen or alkanoyloxy;
to produce a compound of the formula 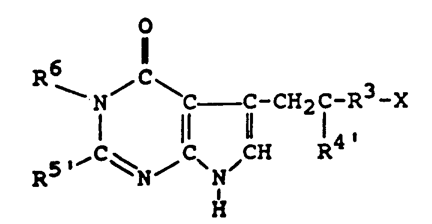 in which X, R^{2'}, R³, R^{4'}S R^{5'} and R⁶ are as defined above;
and when X = COOR^{2'}, coupling with a protected glutamic acid to form a compound where 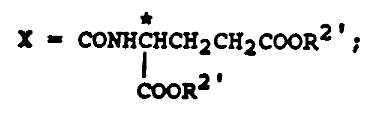 and removing any protecting groups.

13. The process according to claim 12 for the preparation of N-{4-[2-(4-hydroxy-6-aminopyrrolo[2,3-d]pyrimidin-3-yl)ethyl]benzoyl}-L-glutamic acid and the pharmaceutically acceptable salts thereof.

14. A pharmaceutical composition comprising as the active principle a compound of claims 1-11 in combination with a pharmaceutically acceptable carrier.

15. A pharmaceutical composition according to claim 14 wherein the active principle is N-{4-[2-(4-hydroxy-6-aminopyrrolo-[2.3-d]pyridin-3-yl)ethyl]benzoyl}-L-glutamic acid.

16. The use of the compound of claims 1-11 for the preparation of a medicament useful for combatting neoplastic growth in a mammal through inhibition of thymidylate synthetase.

## Claims (Claims for the following Contracting State(s): ES, GR)

1. A process for the preparation of compounds of the formula 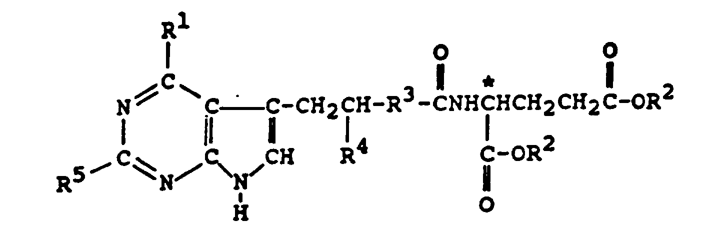 in which:
R¹ is -OH or -NH₂;
R is hydrogen or a pharmaceutically acceptable cation;
R³ is 1,4-phenylene or 1,3-phenylene unsubstituted or substituted with chloro, fluoro, methyl, methoxy, or trifluoromethyl; thienediyl unsubstituted or substituted with chloro, fluoro, methyl, methoxy, or trifluoromethyl; cyclohexanediyl; or a straight or branched divalent aliphatic group of from 2 to 4 carbon atoms;
R⁴ is hydrogen; and
R⁵ is hydrogen, alkyl of 1 to 6 carbon atoms, or amino; and
the configuration about the carbon atom designated * is S;
which comprises catalytically hydrogenating a compound of the formula 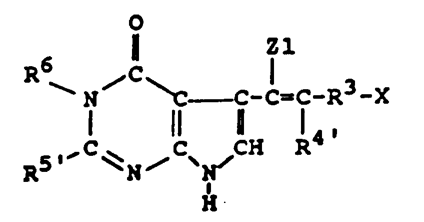 in which:
X is COOR^{2'}; or 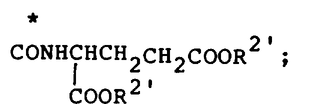
Z¹ is hydrogen, or Z¹ taken together with R^{4'} is a carbon-carbon bond;
R^{2'} is hydrogen or a carboxy protecting group;
R³ is as defined above;
R^{4'} when taken independently of Z¹, is hydrogen;
R^{5'} is hydrogen, alkyl of 1 to 6 carbon atoms, amino, or an amino protecting group; and
R⁶ is hydrogen or alkanoyloxy;
to produce a compound of the formula 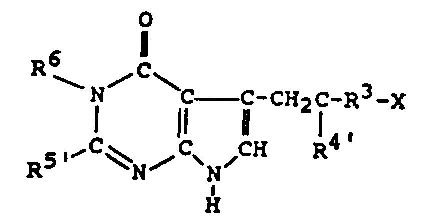
in which X, R^{2'}, R³, R^{4'}, R^{5'} and R⁶ are as defined above;
and when X = COOR^{2'}, coupling with a protected glutamic acid to form a compound where 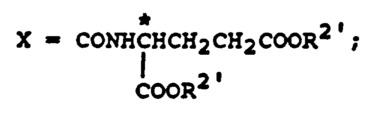 and removing any protecting groups.

2. The process according to claim 1 for the preparation of N-{4-[2-(4-hydroxy-6-aminopyrrolo[2,3-d]pyrimidin-3-yl)ethyl]benzoyl}-L-glutamic acid and the pharmaceutically acceptable salts thereof.

## Patentansprüche (Patentansprüche für folgende(n) Vertragsstaat(en): DE, GB, FR, IT, NL, SE, LI, CH, BE, AT, LU, DK)

1. Verbindung der Formel 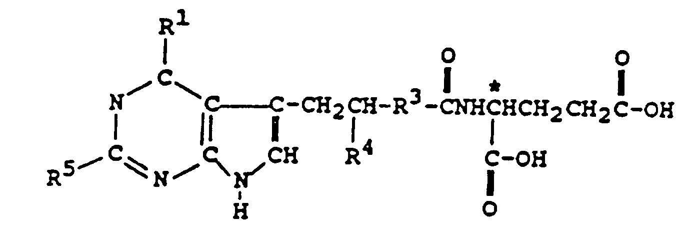 worin bedeuten:
R¹ -OH oder -NH₂;
R³ 1,4-Phenylen oder 1,3-Phenylen, das unsubstituiert oder substituiert ist durch Chlor, Fluor, Methyl, Methoxy oder Trifluoromethyl; Thiendiyl, das unsubstituiert oder substituiert ist durch Chlor, Fluor, Methyl, Methoxy oder Trifluoromethyl; Cyclohexandiyl; oder eine unverzeigte oder verzweigte divalente aliphatische Gruppe mit 2 bis 4 Kohlenstoffatomen;
R⁴ Wasserstoff; und
R⁵ Wasserstoff, Alkyl mit 1 bis 6 Kohlenstoffatomen oder Amino;
wobei die Konfiguration um das mit * bezeichnete Kohlenstoffatom eine S-Konfiguration ist,
und ihre pharmazeutisch akzeptablen Salze.

2. Verbindung nach Anspruch 1, worin bedeuten: R¹ -OH; R³ 1,4-Phenylen; R⁴ Wasserstoff und R⁵ Wasserstoff.

3. Verbindung nach Anspruch 1, worin bedeuten: R¹ -OH; R³ 1,4-Phenylen; R⁴ Wasserstoff und R⁵ Methyl.

4. Verbindung nach Anspruch 1, worin bedeuten: R¹ -OH; R³ Thiendiyl; R⁴ Wasserstoff und R⁵ Wasserstoff.

5. Verbindung nach Anspruch 1, worin bedeuten: R¹ -OH; R³ Thiendiyl; R⁴ Wasserstoff und R⁵ Methyl.

6. Verbindung nach Anspruch 1, bei der es sich handelt um N-{4-[2-(4-Hydroxypyrrolo[2,3-d]pyrimidin-3-yl)ethyl]benzoyl}-L-glutaminsäure und
N-{4-[2-(4-Hydroxy-6-methylpyrrolo[2,3-d]pyrimidin-3-yl)ethyl]benzoyl}-L-glutaminsäure.

7. Verbindung der Formel 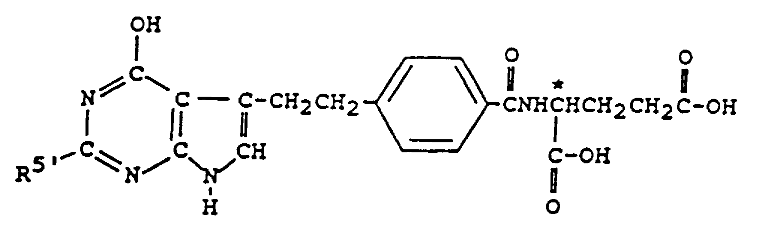 worin R^{5'} für Amino oder Alkanoylamino mit 1 bis 12 Kohlenstoffatomen steht, wobei die Konfiguration um das mit * bezeichnete Kohlenstoffatom eine S-Konfiguration ist, und ihre pharmazeutisch akzeptablen Salze.

8. Verbindung nach Anspruch 7, bei der es sich handelt um N-{4-[2-(4-Hydroxy-6-aminopyrrolo[2,3-d]pyrimidin-3-yl)ethyl]benzoyl}-L-glutaminsäure und ihre pharmazeutisch akzeptablen Salze.

9. Verbindung der Formel 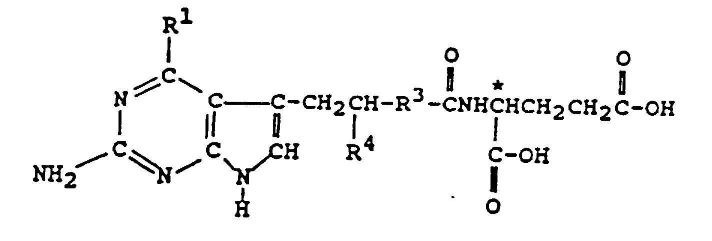 worin bedeuten:
R¹ -OH oder -NH₂;
R³ Thiendiyl, das unsubstituiert oder substituiert ist durch Chlor, Fluor, Methyl, Methoxy oder Trifluoromethyl; Cyclohexandiyl; oder eine unverzeigte oder verzweigte divalente aliphatische Gruppe mit 2 bis 4 Kohlenstoffatomen;
R⁴ Wasserstoff;
wobei die Konfiguration um das mit * bezeichnete Kohlenstoffatom eine S-Konfiguration ist, und ihre pharmazeutisch akzeptablen Salze.

10. Verbindung nach Anspruch 9, worin bedeuten: R¹ -OH; R³ Thiendiyl und R⁴ Wasserstoff.

11. Verbindung nach Anspruch 9, bei der es sich handelt um N-{5-[2-(4-Hydroxy-6-aminopyrrolo[2,3-d]pyrimidin-3-yl)ethyl]thien-2-ylcarbonyl}-L-glutaminsäure; N-{4-[2-(4-Hydroxy-6-aminopyrrolo[2,3-d]pyrimidin-3-yl)ethyl]thien-2-ylcarbonyl}-L-glutaminsäure oder N-{5-[2-(4-Hydroxy-6-aminopyrrolo[2,3-d]pyrimidin-3-yl)ethyl]thien-3-ylcarbonyl}-L-glutaminsäure.

12. Verfahren zur Herstellung von Verbindungen der Formel 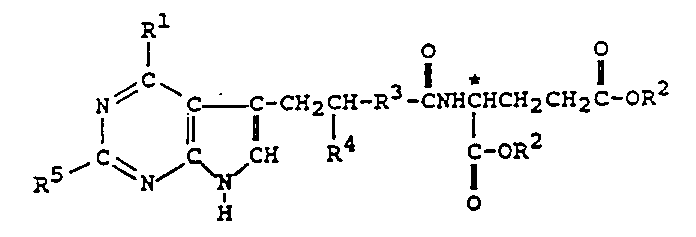 worin bedeuten:
R¹ -OH oder -NH₂;
R Wasserstoff oder ein pharmazeutisch akzeptables Kation;
R³ 1,4-Phenylen oder 1,3-Phenylen, das unsubstituiert oder substituiert ist durch Chlor, Fluor, Methyl, Methoxy oder Trifluoromethyl; Thiendiyl, das unsubstituiert oder substituiert ist durch Chlor, Fluor, Methyl, Methoxy oder Trifluoromethyl; Cyclohexandiyl; oder eine unverzeigte oder verzweigte divalente aliphatische Gruppe mit 2 bis 4 Kohlenstoffatomen;
R⁴ Wasserstoff;
R⁵ Wasserstoff, Alkyl mit 1 bis 6 Kohlenstoffatomen oder Amino;
wobei die Konfiguration um das mit * bezeichnete Kohlenstoffatom eine S-Konfiguration ist;
das umfaßt die katalytische Hydrierung einer Verbindung der Formel 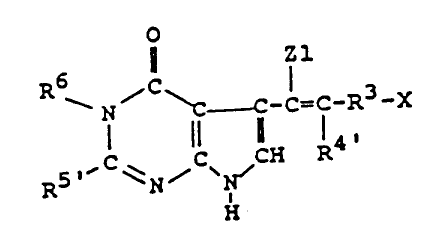 worin bedeuten:
X COOR^{2'}; oder 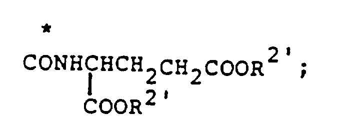
Z¹ Wasserstoff oder Z¹ zusammengenommen mit R^{4'} eine Kohlenstoff-Kohlenstoff-Bindung;
R^{2'} Wasserstoff oder eine Carboxy-Schutzgruppe;
R³ wie oben definiert ist;
R^{4'}, einzeln und unabhängig von Z¹, Wasserstoff;
R⁵' Wasserstoff, Alkyl mit 1 bis 6 Kohlenstoffatomen, Amino oder eine Amino-Schutzgruppe; und
R⁶ Wasserstoff oder Alkanoyloxy;
unter Bildung einer Verbindung der Formel 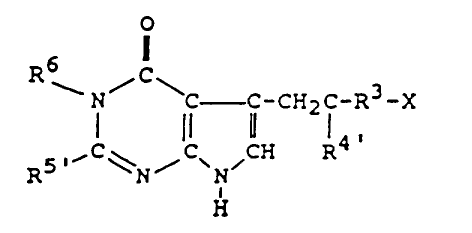 worin X, R^{2'}, R³, R^{4'}, R^{5'} und R⁶ wie oben definiert sind; und
dann, wenn X = COOR^{2'}, das Kuppeln mit einer geschützten Glutaminsäure unter Bildung einer Verbindung, in der 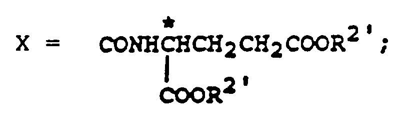 und
das Entfernen irgendwelcher Schutzgruppen.

13. Verfahren nach Anspruch 12 zur Herstellung von N-{4-[2-(4-Hydroxy-6-aminopyrrolo-[2,3-d]pyrimidin-3-yl)ethyl]-benzoyl}-L-glutaminsäure und ihrer pharmazeutisch akzeptablen Salze.

14. Pharmazeutische Zusammensetzung, die als aktives Prinzip (Wirkstoff) eine Verbindung der Ansprüche 1 bis 11 in Kombination mit einem pharmazeutisch akzeptablen Träger enthält.

15. Pharmazeutische Zusammensetzung nach Anspruch 14, in der das aktive Prinzip (der Wirkstoff) N-{4-[2-(4-Hydroxy-6-aminopyrrolo-[2,3-d]pyridin-3-yl)ethyl]benzoyl}-L-glutaminsäure ist.

16. Verwendung der Verbindung nach den Ansprüchen 1 bis 11 zur Herstellung eines Arzneimittels, das zur Bekämpfung von neoplastischem Wachstum in einem Säugetier durch Inhibierung der Thymidilat-Synthetase geeignet ist.

## Patentansprüche (Patentansprüche für folgende(n) Vertragsstaat(en): ES, GR)

1. Verfahren zur Herstellung von Verbindungen der Formel 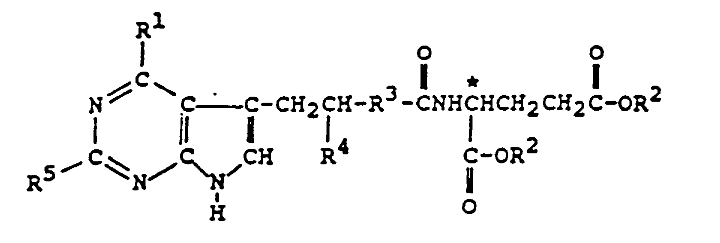 worin bedeuten:
R¹ -OH oder -NH₂;
R Wasserstoff oder ein pharmazeutisch akzeptables Kation;
R³ 1,4-Phenylen oder 1,3-Phenylen, das unsubstituiert oder substituiert ist durch Chlor, Fluor, Methyl, Methoxy oder Trifluoromethyl; Thiendiyl, das unsubstituiert oder substituiert ist durch Chlor, Fluor, Methyl, Methoxy oder Trifluoromethyl; Cyclohexandiyl; oder eine unverzeigte oder verzweigte divalente aliphatische Gruppe mit 2 bis 4 Kohlenstoffatomen;
R⁴ Wasserstoff;
R⁵ Wasserstoff, Alkyl mit 1 bis 6 Kohlenstoffatomen oder Amino;
wobei die Konfiguration um das mit * bezeichnete Kohlenstoffatom eine S-Konfiguration ist;
das umfaßt die katalytische Hydrierung einer Verbindung der Formel 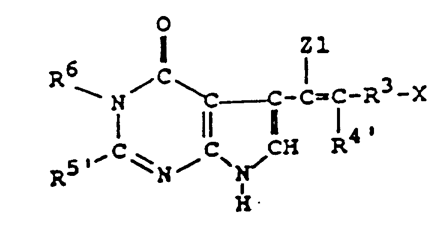 worin bedeuten:
X COOR; oder 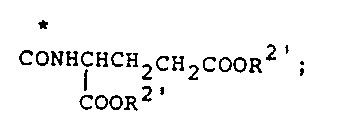
Z¹ Wasserstoff oder Z¹ zusammengenommen mit R^{4'} eine Kohlenstoff-Kohlenstoff-Bindung;
R^{2'} Wasserstoff oder eine Carboxy-Schutzgruppe;
R³ wie oben definiert ist;
R^{4'}, einzeln und unabhängig von Z¹, Wasserstoff;
R^{5'} Wasserstoff, Alkyl mit 1 bis 6 Kohlenstoffatomen, Amino oder eine Amino-Schutzgruppe; und
R⁶ Wasserstoff oder Alkanoyloxy;
unter Bildung einer Verbindung der Formel 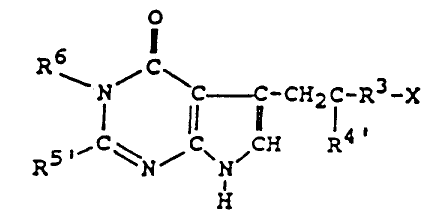 worin X, R^{2'}, R³, R^{4'}, R^{5'} und R⁶ wie oben definiert sind; und
dann, wenn X = COOR^{2'}, das Kuppeln mit einer geschützten Glutaminsäure unter Bildung einer Verbindung, in der 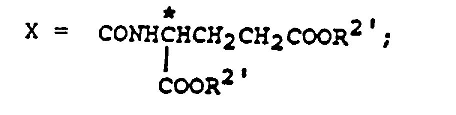 und
das Entfernen irgendwelcher Schutzgruppen.

2. Verfahren nach Anspruch 1 zur Herstellung von N-{4-[2-(4-Hydroxy-6-aminopyrrolo-[2,3-d]pyrimidin-3-yl)ethyl]-benzoyl}-L-glutaminsäure und ihrer pharmazeutisch akzeptablen Salze.

## Revendications (Revendications pour l'(les) Etat(s) contractant(s) suivant(s): DE, GB, FR, IT, NL, SE, LI, CH, BE, AT, LU, DK)

1. Composé de formule : 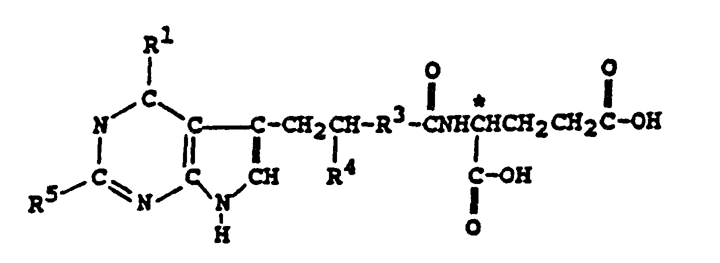 dans laquelle
R¹ représente -OH ou -NH₂ ;
R³ représente un groupe 1,4-phénylène ou 1,3-phénylène non substitué ou substitué par un atome de chlore, de fluor, un groupe méthyle, méthoxy, ou trifluorométhyle ; un groupe thiènediyle non substitué ou substitué par un atome de chlore, de fluor, un groupe méthyle, méthoxy, ou trifluorométhyle ; un groupe cyclohexanediyle ; ou un groupe aliphatique divalent linéaire ou ramifié comprenant de 2 à 4 atomes de carbone ;
R⁴ représente un atome d'hydrogène ; et
R⁵ représente un atome d'hydrogène, un groupe alkyle comprenant de 1 à 6 atomes de carbone, ou un groupe amino; la configuration correspondant à l'atome de carbone désigné par * est la configuration S ; et
et ses sels acceptables du point de vue pharmaceutique.

2. Composé selon la revendication 1, dans lequel R¹ représente -OH ; R³ représente un groupe 1,4-phénylène ; R⁴ représente un atome d'hydrogène et R⁵ représente un atome d'hydrogène.

3. Composé selon la revendication 1, dans lequel R¹ représente -OH ; R³ représente un groupe 1,4-phénylène; R⁴ représente un atome d'hydrogène et R⁵ représente un groupe méthyle.

4. Composé selon la revendication 1, dans lequel R¹ représente -OH ; R³ représente un groupe thiènediyle ; R⁴ représente un atome d'hydrogène et R⁵ représente un atome d'hydrogène.

5. Composé selon la revendication 1, dans lequel R¹ représente -OH ; R³ représente un groupe thiènediyle ; R⁴ représente un atome d'hydrogène et R5 représente un groupe méthyle.

6. Composé selon la revendication 1, qui est l'acide N-{4-[2-(4-hydroxypyrrolo[2,3-d]pyrimidin-3-yl)éthyl]ben zoyl}-L-glutamique, et l'acide N-{4-[2-(4-hydroxy-6-méthylpyrrolo[2,3-d]pyrimidin-3-yl) éthyl]benzoyl}-L-glutamique.

7. Composé de formule : 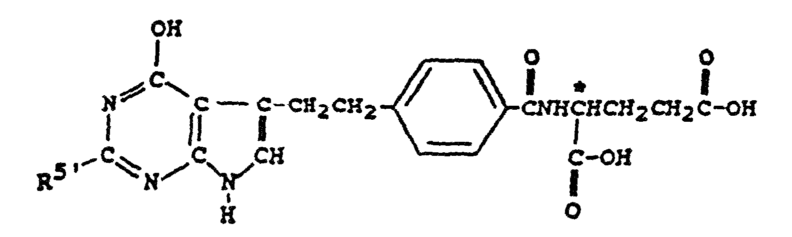 dans laquelle R^{5'} est un groupe amino ou alcanoylamino comprenant de 1 à 12 atomes de carbone, la configuration correspondant à l'atome de carbone désigné par * est la configuration S, et ses sels acceptables du point de vue pharmaceutique.

8. Composé de la revendication 7, qui est l'acide N-{4-[2-(4-hydroxy-6-aminopyrrolo[2,3-d]pyrimidin-3-yl)éthyl]benzoyl}-L-glutamique et ses sels acceptables du point de vue pharmaceutique.

9. Composé de formule : 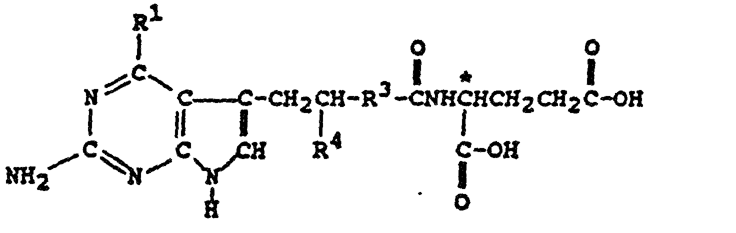 dans laquelle
R¹ représente -OH ou -NH₂ ;
R³ représente un groupe thiènediyle non substitué ou substitué par un atome de chlore, de fluor, un groupe méthyle, méthoxy, ou trifluorométhyle ; un groupe cyclohexanediyle ; ou un groupe aliphatique divalent linéaire ou ramifié comprenant de 2 à 4 atomes de carbone; R⁴ représente un atome d'hydrogène ; et
la configuration correspondant à l'atome de carbone désigné par * est la configuration S ; et et ses sels acceptables du point de vue pharmaceutique.

10. Composé selon la revendication 9, dans lequel R¹ représente -OH ; R³ représente un groupe thiènediyle, et R⁴ représente un atome d'hydrogène.

11. Composé selon la revendication 9, qui est l'acide N-{5-[2-(4-hydroxy-6-aminopyrrolo[2,3-d]pyrimidin-3-yl) éthyl]thièn-2-ylcarbonyl}-L-glutamique ; l'acide N-{4-[2-(4-hydroxy-6-aminopyrrolo[2,3-d]pyrimidin-3-yl) éthyl]thièn-2-ylcarbonyl}}-L-glutamique ; ou l'acide N-{5-[2-(4-hydroxy-6-aminopyrrolo[2,3-d]pyrimidin-3-yl) éthyl]thièn-2-ylcarbonyl}-L-glutamique.

12. Procédé de préparation de composés de formule : 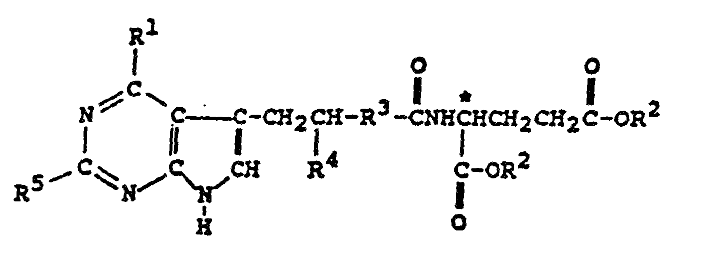 dans laquelle
R¹ représente -OH ou -NH₂ ;
R représente un atome d'hydrogène ou un cation acceptable du point de vue pharmaceutique ;
R³ représente un groupe 1,4-phénylène ou 1,3-phénylène non substitué ou substitué par un atome de chlore, de fluor, un groupe méthyle, méthoxy, ou trifluorométhyle ; un groupe thiènediyle non substitué ou substitué par un atome de chlore, de fluor, un groupe méthyle, méthoxy, ou trifluorométhyle ; un groupe cyclohexanediyle ; ou un groupe aliphatique divalent linéaire ou ramifié comprenant de 2 à 4 atomes de carbone ;
R⁴ représente un atome d'hydrogène ; et
R⁵ représente un atome d'hydrogène, un groupe alkyle comprenant de 1 à 6 atomes de carbone, ou un groupe amino; et
la configuration correspondant à l'atome de carbone désigné par * est la configuration S ;
qui comprend l'hydrogénation catalytique d'un composé de formule 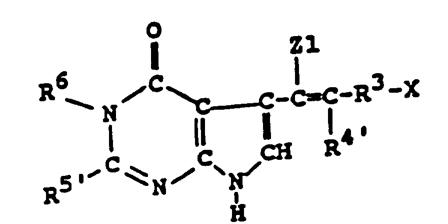 dans laquelle
X représente COOR^{2'} ; ou 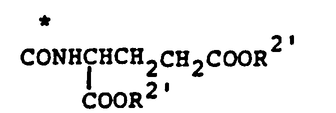
Z¹ représente un atome d'hydrogène, ou Z¹ et R^{4'} pris ensemble représentent une liaison carbone-carbone ;
R^{2'} représente un atome d'hydrogène ou un groupe protecteur de groupe carboxy ;
R³ est tel que défini ci-dessus ;
R^{4'}, lorsqu'il est pris indépendamment de Z¹, représente un atome d'hydrogène ;
R^{5'} représente un atome d'hydrogène, un groupe alkyle comprenant de 1 à 6 atomes de carbone, un groupe amino, ou un groupe protecteur de groupe amino ; et
R⁶ représente un atome d'hydrogène ou un groupe alcanoyloxy;
pour produire un composé de formule 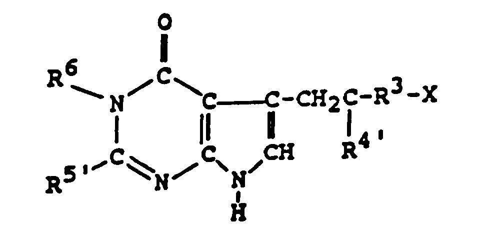
dans laquelle X, R^{2'}, R^{3'}, R^{4'}, R^{5'} et R⁶ sont tels que définis ci-dessus ;
et lorsque X = COOR^{2'}, le couplage avec un acide glutamique protégé pour former un composé dans lequel 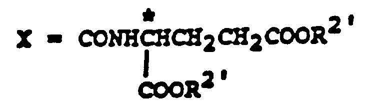 et l'élimination de tous les groupes protecteurs.

13. Procédé selon la revendication 12 pour la préparation d'acide N-{4-[2-(4-hydroxy-6-aminopyrrolo[2,3-d]pyrimidin-3-yl) éthyl]benzoyl}-L-glutamique, et de ses sels acceptables du point de vue pharmaceutique.

14. Composition pharmaceutique comprenant comme principe actif un composé selon les revendications 1 à 11 en combinaison avec un véhicule acceptable du point de vue pharmaceutique.

15. Composition pharmaceutique selon la revendication 14, dans lequel le principe actif est l'acide N-{4-[2-(4-hydroxy-6-aminopyrrolo[2,3-d]pyridin-3-yl)éthyl]benzoyl}-L-glutamique.

16. Utilisation du composé des revendications 1 à 11 pour la préparation d'un médicament utile pour combattre la croissance néoplasique chez un mammifère, par l'inhibition de la thymidilate synthétase.

## Revendications (Revendications pour l'(les) Etat(s) contractant(s) suivant(s): ES, GR)

1. Procédé de préparation de composés de formule : 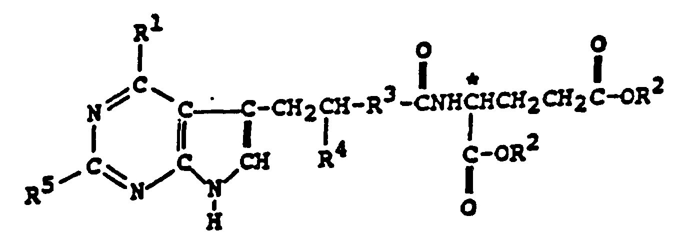 dans laquelle
R¹ représente -OH ou -NH₂ ;
R représente un atome d'hydrogène ou un cation acceptable du point de vue pharmaceutique ;
R³ représente un groupe 1,4-phénylène ou 1,3-phénylène non substitué ou substitué par un atome de chlore, de fluor, un groupe méthyle, méthoxy, ou trifluorométhyle ; un groupe thiènediyle non substitué ou substitué par un atome de chlore, de fluor, un groupe méthyle, méthoxy, ou trifluorométhyle ; un groupe cyclohexanediyle ; ou un groupe aliphatique divalent linéaire ou ramifié comprenant de 2 à 4 atomes de carbone ;
R⁴ représente un atome d'hydrogène ; et
R⁵ représente un atome d'hydrogène, un groupe alkyle comprenant de 1 à 6 atomes de carbone, ou un groupe amino; et
la configuration correspondant à l'atome de carbone désigné par * est la configuration S ;
qui comprend l'hydrogénation catalytique d'un composé de formule 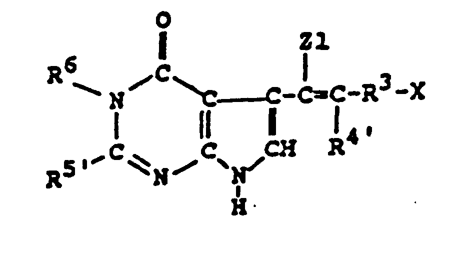 dans laquelle
X représente COOR^{2'} ; ou 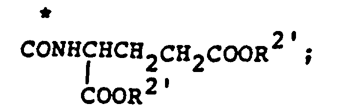
Z¹ représente un atome d'hydrogène, ou Z¹ et R^{4'} pris ensemble représentent une liaison carbone-carbone ;
R^{2'} représente un atome d'hydrogène ou un groupe protecteur de groupe carboxy ;
R³ est tel que défini ci-dessus ;
R^{4'}, lorsqu'il est pris indépendamment de Z¹, représente un atome d'hydrogène ;
R^{5'} représente un atome d'hydrogène, un groupe alkyle comprenant de 1 à 6 atomes de carbone, un groupe amino, ou un groupe protecteur de groupe amino ; et
R⁶ représente un atome d'hydrogène ou un groupe alcanoyloxy;
pour produire un composé de formule : 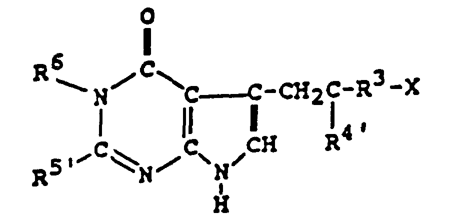
dans laquelle X, R^{2'}, R^{3'}, R^{4'}, R^{5'} et R⁶ sont tels que définis ci-dessus ;
et lorsque X = COOR^{2'}, le couplage avec un acide glutamique protégé pour former un composé dans lequel 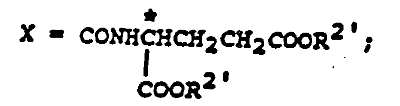 et l'élimination de tous les groupes protecteurs

2. Procédé selon la revendication 1 pour la préparation d'acide N-{4-[2-(4-hydroxy-6-aminopyrrolo[2,3d]pyrimidin-3-yl)éthyl]benzoyl}-L-glutamique, et ses sels acceptables du point de vue pharmaceutique.
